(19) Europäisches Patentamt
European Patent Office
Office européen des brevets

(11) **EP 4 360 661 A1**

(12) **EUROPEAN PATENT APPLICATION**

(43) Date of publication:
**01.05.2024 Bulletin 2024/18**

(21) Application number: **22203328.4**

(22) Date of filing: **24.10.2022**

(51) International Patent Classification (IPC):
**A61K 51/12** (2006.01)  **A61K 103/10** (2006.01)
**B82Y 15/00** (2011.01)  **A61P 19/02** (2006.01)
**A61P 35/00** (2006.01)

(52) Cooperative Patent Classification (CPC):
**A61K 51/1251; A61P 19/02; A61P 35/00;
B82Y 30/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Universität Regensburg
93053 Regensburg (DE)**

(72) Inventors:
• **Moosbauer, Jutta
93053 Regensburg (DE)**
• **Hellwig, Dirk
93053 Regensburg (DE)**
• **Stahl, Monika
92431 Neunburg vorm Wald (DE)**
• **Göpferich, Achim
93161 Sinzing (DE)**

(74) Representative: **Engelhard, Markus
Boehmert & Boehmert
Anwaltspartnerschaft mbB
Pettenkoferstrasse 22
80336 München (DE)**

(54) **NANOPARTICLES FOR MEDICAL IMAGING AND TREATMENT**

(57) The present invention relates to a nanoparticle comprising a block copolymer comprising or consisting of a hydrophobic block and a hydrophilic block. The present invention further relates to a pharmaceutical composition comprising a nanoparticle. The present invention also relates to the nanoparticle or pharmaceutical composition for use in medicine; preferably for use in a method of preventing, treating, monitoring, and/or diagnosing a disease. Furthermore, the present invention relates to a method of preparing a nanoparticle and to a kit.

**Figure 6**

**Description**

FIELD OF THE INVENTION

The present invention relates to a nanoparticle comprising a block copolymer comprising or consisting of a hydrophobic block and a hydrophilic block. The present invention further relates to a pharmaceutical composition comprising a nanoparticle. The present invention also relates to the nanoparticle or pharmaceutical composition for use in medicine; preferably for use in a method of preventing, treating, monitoring, and/or diagnosing a disease. Furthermore, the present invention relates to a method of preparing a nanoparticle and to a kit.

BACKGROUND OF THE INVENTION

In case of breast and prostate carcinoma, skin tumors and many other types of cancer, the removal and histological examination of the first lymph nodes in the lymphatic drainage area of the primary tumor, the so-called sentinel lymph nodes (SLNs), is essential for precise diagnosis of the disease and correct choice of therapy. SLN biopsy is used to investigate whether or not tumor cells are already present in the first lymph nodes, which in turn provides information about the metastatic stage of the tumor. The SLNs are localized and removed during surgery, often at the same time the primary tumor is removed. For this, a radiotracer - a radiolabelled colloid or macromolecule - is injected into the area surrounding the tumor, which then drains to the SLNs and is detected using a hand-held gamma probe. The ideal SLN detection agent has the following properties: 1) rapid clearance from injection site, 2) selective uptake into the lymphatic system and high retention in sentinel lymph nodes, 3) low uptake in distal lymph nodes, 4) easily detectable signal intensity and 5) biodegradation/elimination after signal detection. A radiotracer combining all these properties enables high accuracy of SLN detection with minimal surgical burden and radiation exposure for patients and medical staff.

Various radiotracers labelled with technetium-99m ($^{99m}$Tc) are currently used for SLN biopsy. $^{99m}$Tc offers many advantages in terms of use in lymphoscintigraphy. The nuclide has a short half-life of 6.02 hours, is relatively cheap and readily available, shows low absorbed-dose burden to patients, and finally has almost no influence on the biochemical properties of the carrier. [$^{99m}$Tc]Tc-sulfur colloid ([$^{99m}$Tc]Tc-SC), [$^{99m}$Tc]Tc-human serum albumin ([$^{99m}$Tc]Tc-HSA) and [$^{99m}$Tc]Tc-diethylenetriaminepentaacetic acid (DTPA)-mannosyl-dextran ([$^{99m}$Tc]Tc-tilmanocept) are the most commonly used diagnostic agents for SLN imaging at the moment. However, each of them has disadvantages. For example, [$^{99m}$Tc]Tc-SCs show a wide size distribution with many large particles, making them not ideal for SLN detection as they remain largely at the injection site. Therefore, the [$^{99m}$Tc]Tc-sulfur colloids are filtered after labelling to achieve the appropriate size und size distribution, but this in turn leads to the loss of a significant amount of radioactive label. [$^{99m}$Tc]Tc-HSA is problematic due to its allergenic potential and its classification as a blood product, which implies time-consuming regulatory obligations such as extended patient information and batch documentation inspected by legal authorities. Finally, [$^{99m}$Tc]Tc-tilmanocept is a very expensive radiotracer and DTPA is required as chelating agent to achieve sufficient labelling efficiency.

Thus, improved diagnostic agents, e.g. radiotracers, are needed. Particularly, there remains the need for detection agents for medical imaging and radioguided surgery. Furthermore, there remains the need for efficient methods of providing detection agents for medical imaging and radioguided surgery. Moreover, there remains the need for detection agents with narrow size distribution, e.g. suitable for sentinel lymph node imaging. There also remains the need for improving medical imaging and radioguided surgery. Moreover, there remains the need for agents that allow to prevent or treat diseases with high precision, e.g. malignant diseases, diseases associated with the lymphatic system, diseases in a preformed body cavity, and/or joint diseases. For example, there remains the need for agents that allow for localized irradiation, such as for use in radiosynovectomy, radiotympanosinuorthesis, instillation therapy of cystic craniopharyngeomas, and sclerotherapy of lymphocele.

SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a nanoparticle comprising a block copolymer comprising or consisting of a hydrophobic block and a hydrophilic block;

wherein said nanoparticle comprises a core and a surface, wherein said core comprises said hydrophobic block and wherein said hydrophilic block is oriented towards said surface; wherein said hydrophobic block comprises or consists of a hydrophobic polymer, preferably poly(lactic-co-glycolic acid); and
wherein said hydrophilic block comprises alkoxy polyethylene glycol, preferably selected from $C_1$-$C_4$ alkoxy polyethylene glycol, more preferably methoxy polyethylene glycol.

In one embodiment, said nanoparticle comprises a label, optionally a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{124}$I, $^{125}$I, $^{131}$I, $^{169}$Er, $^{177}$Lu, $^{186}$Re, $^{188}$Re, $^{198}$Au, and any combination thereof; even more preferably $^{99m}$Tc.

In one embodiment, said block copolymer is a diblock copolymer or triblock copolymer, preferably a diblock copolymer, more preferably a methoxy-poly(ethylene glycol)-block-poly(lactide-co-glycolide).

In one embodiment, said core further comprises a polymer different from said block copolymer, said polymer preferably comprising or consisting of poly(lactic-co-glycolic acid).

In one embodiment, said nanoparticle has a size in a range of from 5 nm to 200 nm, preferably in a range of from 10 nm to 60 nm, more preferably in a range of from 10 nm to 50 nm, even more preferably in a range of from 13.5 nm to 30 nm.

In one embodiment, said nanoparticle comprises a targeting agent, preferably a lymph node targeting agent and/or a tumor targeting agent;
wherein, preferably, said targeting agent comprises or consists of a ligand, a moiety-binding motif, an enzyme inhibitor, and/or an antigen-binding peptide.

In a further aspect, the present invention relates to a pharmaceutical composition comprising a nanoparticle, as defined herein, and a pharmaceutically acceptable excipient.

In a further aspect, the present invention relates to a nanoparticle, as defined herein, or pharmaceutical composition, as defined herein, for use in medicine;

preferably for use in a method of preventing, treating, monitoring, and/or diagnosing a disease, optionally selected from a malignant disease, a disease associated with the lymphatic system, a disease in a preformed body cavity, and a joint disease;
more preferably for use in medical imaging, radioguided surgery, and/or radionuclide therapy, preferably in scintigraphy such as lymphoscintigraphy, single-photon emission computed tomography (SPECT), positron emission tomography (PET), and/or in radiosynovectomy.

In a further aspect, the present invention relates to a method of preparing a nanoparticle, preferably a nanoparticle as defined herein, comprising

a) providing a block copolymer comprising or consisting of a hydrophobic block and a hydrophilic block,
wherein said hydrophobic block comprises or consists of a hydrophobic polymer, preferably poly(lactic-co-glycolic acid), and wherein said hydrophilic block comprises alkoxy polyethylene glycol, preferably selected from $C_1$-$C_4$ alkoxy polyethylene glycol, more preferably methoxy polyethylene glycol;
b) dissolving said block copolymer in an organic solvent, preferably selected from acetonitrile, N,N-dimethylformamide, acetone, tetrahydrofuran, dimethyl sulfoxide, and any combination thereof, more preferably acetonitrile, thereby obtaining a polymer solution;
c) optionally, adding a polymer different from said block copolymer, said polymer preferably comprising or consisting of poly(lactic-co-glycolic acid), to said polymer solution;
d) contacting, particularly mixing, said polymer solution obtained in step b) or step c) with an aqueous phase, preferably using a microfluidic system;
e) obtaining nanoparticle(s), preferably by self-assembly, more preferably by nanoprecipitation;
said nanoparticle(s) optionally having a size in a range of from 5 nm to 200 nm, preferably in a range of from 10 nm to 60 nm, more preferably in a range of from 10 nm to 50 nm, even more preferably in a range of from 13.5 nm to 30 nm;
f) optionally, lyophilizing said nanoparticle(s) to obtain lyophilized nanoparticle(s).

In one embodiment, the method comprises step c) of adding a polymer different from said block copolymer, said polymer preferably comprising or consisting of poly(lactic-co-glycolic acid), to said polymer solution;
wherein, preferably, said block copolymer and said polymer different from said block copolymer have a weight ratio of about 7:3 (m/m).

In one embodiment, said method further comprises labelling, preferably radioactively labelling, said nanoparticle(s) obtained in step e) or said lyophilized nanoparticle(s) obtained in step f); said labelling preferably comprising

i) providing a label, optionally a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{124}$I, $^{125}$I, $^{131}$I, $^{169}$Er, $^{177}$Lu, $^{186}$Re, $^{188}$Re, $^{198}$Au, and any combination thereof; even more preferably $^{99m}$Tc, preferably $^{99m}$Tc pertechnetate, more preferably a $^{99m}$Tc pertechnetate solution; and mixing said label, preferably $^{99m}$Tc pertechnetate, with said nanoparticle(s) obtained in step e) and/or said lyophilized nanoparticle(s) obtained in step f);

ii) optionally, adding a reducing agent, preferably $SnCl_2$, thereby obtaining a reduced label, preferably reduced $^{99m}$Tc pertechnetate;

iii) optionally, adding a stabilizer, preferably ascorbic acid and/or ethanol;

iv) allowing said label, optionally said reduced label obtained in step ii), preferably reduced $^{99m}$Tc pertechnetate obtained in step ii), to label said nanoparticle(s);

v) obtaining labelled nanoparticle(s);

wherein, preferably, said labelling is performed in the absence of oxygen;

wherein, optionally, said labelling is performed at a pH of about pH 4.5 to about pH 7.5, preferably at a pH of about pH 4.5 to about pH 5.5, more preferably at about pH 5, and/or

for about 1 min to about 6 hours, preferably about 10 min to about 1.5 hours, more preferably about 10 min to about 20 min, and/or

at a temperature of about 15°C to about 40°C, preferably at room temperature.

In one embodiment, said mixing in step i) comprises mixing said label, preferably $^{99m}$Tc pertechnetate, with said nanoparticle(s) at a molar ratio of about 1:1, and/or if step ii) is present, said adding in step ii) comprises providing a molar ratio of said reducing agent, preferably $SnCl_2$, to said label, preferably $^{99m}$Tc pertechnetate, in a range of from about 1000:1 to about 100000:1, optionally in a range of from about 3000:1 to about 30000:1.

In one embodiment, said label, preferably $^{99m}$Tc pertechnetate, has a radioactivity, preferably at a time of preparation, in a range of from 4 MBq to 8000 MBq, preferably in a range of from 400 MBq to 2200 MBq, more preferably in a range of from 400 MBq to 1000 MBq.

In one embodiment, said method comprises coupling a targeting agent, preferably a lymph node targeting agent and/or a tumor targeting agent, to said nanoparticle, preferably by coupling said targeting agent to said block copolymer, optionally by coupling said targeting agent to said block copolymer using ethyl(dimethylaminopropyl) carbodiimide and/or N-hydroxysuccinimide.

In one embodiment, said nanoparticle, said label, said block copolymer, and/or said targeting agent are as defined above.

In a further aspect, the present invention relates to a kit comprising, preferably in separate containers,

- a nanoparticle as defined herein, optionally in a lyophilized form; a pharmaceutical composition as defined herein; and/or a nanoparticle prepared by a method as defined herein, optionally in a lyophilized form;
- optionally, a label, such as a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{125}$I, $^{125}$I, $^{131}$I, $^{169}$Er, $^{177}$Lu, $^{186}$Re, $^{188}$Re, $^{198}$Au, and any combination thereof; even more preferably $^{99m}$Tc, preferably $^{99m}$Tc pertechnetate, optionally a $^{99m}$Tc pertechnetate solution, e.g. a solution of sodium pertechnetate;
- optionally, a reducing agent, preferably $SnCl_2$; and
- optionally, a bulking agent, a buffer, a cryoprotectant, a stabilizing agent, and/or a tonicity regulating agent.

In one embodiment, said nanoparticle, said pharmaceutical composition, and/or said label are as defined above.

In a further aspect, the present invention relates to a method of preventing, treating, monitoring, and/or diagnosing a disease, optionally selected from a malignant disease, a disease associated with the lymphatic system, a disease in a preformed body cavity, and a joint disease, comprising administering a nanoparticle, as defined herein, or a pharmaceutical composition, as defined herein, to a patient in need thereof.

In one embodiment, said method comprises or consists of medical imaging, radioguided surgery, and/or radionuclide therapy, preferably scintigraphy such as lymphoscintigraphy, single-photon emission computed tomography (SPECT), positron emission tomography (PET), and/or radiosynovectomy.

In one embodiment, said administering comprises administering an effective amount of a nanoparticle, as defined herein, and/or a pharmaceutical composition, as defined herein, to a patient in need thereof.

In a further aspect, the present invention relates to a use of a nanoparticle, as defined herein, or of a pharmaceutical composition, as defined herein, for the manufacture of a medicament, preferably a medicament for preventing, treating, monitoring, and/or diagnosing a disease, optionally selected from a malignant disease, a disease associated with the lymphatic system, a disease in a preformed body cavity, and a joint disease; e.g. a medicament for medical imaging, radioguided surgery, and/or radionuclide therapy, preferably scintigraphy such as lymphoscintigraphy, single-photon emission computed tomography (SPECT), positron emission tomography (PET), and/or radiosynovectomy.

DETAILED DESCRIPTION

Sentinel lymph node (SLN) biopsy is one of the most frequently used diagnostic methods in everyday clinical work. SLN examination determines the choice of treatment in the therapy of many cancer diseases. Therefore, the need for good SLN detection agents with a high accuracy is enormous. [99mTc]Tc-Human serum albumin, [99mTc]Tc-sulfur colloids and [99mTc]Tc-tilmanocept are the most commonly used products at the moment, but they all have disadvantages such as time-consuming handling, poor detection accuracy due to wide size distribution, and high costs, respectively. The invention provides a new SLN detection agent based on polymeric nanoparticles to overcome the limitations of the currently used radiotracers. Methoxy-terminated poly(ethylene glycol)-poly(lactic-co-glycolic acid) (MeO-PEG-PLGA) nanoparticles were synthesized using microfluidics. The inventors optimized the preparation parameters to finally obtain nanoparticles, particularly nanoparticles with a diameter between 10 and 50 nm - with the majority smaller than 30 nm - as these are ideal size characteristics for SLN imaging. The inventors provide an advantageous method in which subsequent radioactive labelling with $^{99m}$Tc is possible directly without complexing agent. The inventors evaluated optimal conditions for the labelling reaction, e.g. in terms of stannous chloride amount and pH, and finally obtained a product with > 99% radiochemical purity, e.g. about 100% radiochemical purity. Overall, the nanoparticle of the invention, e.g. the [99mTc]Tc-MeO-PEG-PLGA nanoparticle, is an attractive alternative radiotracer that offers ideal size distribution, is not categorized as blood product, is inexpensive and, moreover, does not contain a chelating agent, thereby overcoming the major drawbacks of the current SLN detection agents.

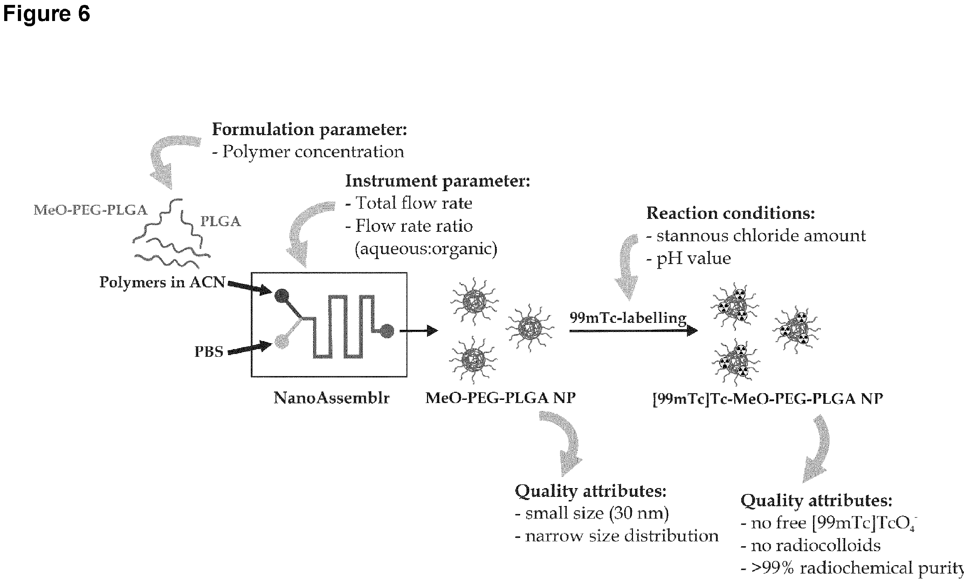

An aim of the invention was to develop a particulate radiotracer based on polymeric nanoparticles (NPs) as an alternative to the existing SLN detection agents (Figure 6). The inventors prepared PEG-PLGA NPs with a PLGA-stabilized core using microfluidics and tested various process parameters to finally obtain small particles with a narrow size distribution that are ideally suited for SLN imaging. Additionally, the inventors optimized labelling, e.g. $^{99m}$Tc-labelling of the particles by testing different reducing agent amounts e.g. stannous chloride amounts and pH values. Finally, the stability of the labelled product was tested by challenging with DTPA and cysteine.

In a first aspect, the present invention relates to a nanoparticle comprising a block copolymer comprising or consisting of a hydrophobic block and a hydrophilic block;

> wherein said nanoparticle comprises a core and a surface, wherein said core comprises said hydrophobic block and wherein said hydrophilic block is oriented towards said surface; wherein said hydrophobic block comprises or consists of a hydrophobic polymer, preferably poly(lactic-co-glycolic acid); and
> wherein said hydrophilic block comprises alkoxy polyethylene glycol, preferably selected from $C_1$-$C_4$ alkoxy polyethylene glycol, more preferably methoxy polyethylene glycol.

In one embodiment, the term "nanoparticle", as used herein, relates to a nanoparticle comprising a hydrophobic polymer and an alkoxy polyethylene glycol. In one embodiment, a nanoparticle is an object with all three external dimensions in the nanoscale, whose longest and shortest axes do not differ significantly, with a significant difference typically being a factor of at least 3. In one embodiment, the nanoparticle comprises a core and a surface, wherein said core comprises said hydrophobic block and optionally further comprises said hydrophilic block or a part of said hydrophilic block; wherein optionally, a part of said hydrophilic block is presented on said surface and/or forms said surface. In one embodiment, the terms "nanoparticle" and "particle" are used interchangeably.

In one embodiment, the hydrophobic block and the hydrophilic block of the block copolymer of the nanoparticle are arranged such that the hydrophobic block is oriented towards a center of the nanoparticle and the hydrophilic block is oriented towards the surface of the nanoparticle. In one embodiment, the nanoparticle comprises a plurality of block copolymer molecules, wherein the plurality of block copolymer molecules are arranged such that the hydrophobic blocks of the block copolymer molecules are comprised by the core of the nanoparticle and/or are oriented towards a center of the nanoparticle, and the hydrophilic blocks of the block copolymer molecules are oriented towards a surface of the nanoparticle. In one embodiment, the term "oriented towards" in the context of the hydrophilic block means that the hydrophilic block has an end which is bound to said hydrophobic block and an end which is not bound to said hydrophobic block, and that the end which is not bound to said hydrophobic block points towards said surface, is part of said surface, and/or forms said surface. In one embodiment, the term "oriented towards" in the context of the hydrophilic block means that the terminal end of the block copolymer comprising the terminal end of the hydrophilic block points towards said surface, is part of said surface, and/or forms said surface. For example, the term "oriented towards" in the context of the hydrophilic block may comprise a scenario in which the block copolymer has a longitudinal extension including a longitudinal extension of said hydrophilic block and a longitudinal extension of said hydrophobic block, wherein a longitudinal axis parallel to said longitudinal extension of said hydrophilic block is about perpendicular to a tangent line of said surface. Furthermore, the term "oriented towards" in the context of the hydrophilic block may comprise a scenario in which a longitudinal extension of said hydrophilic block is about parallel to a radius of said nanoparticle. In one embodiment, the hydrophilic block comprises a terminal end which is not bound to said hydrophobic block and a non-terminal end which is bound to said hydrophobic block. In one embodiment, a terminal end of said hydrophilic block is oriented towards said surface, is part of said surface, and/or forms said surface. In one embodiment, said hydrophilic block is oriented towards said surface such that the terminal end of said hydrophilic block is comprised by said surface. In one embodiment, said hydrophilic block is oriented towards said surface such that said terminal end of said hydrophilic block is comprised by said surface and said non-terminal end is not comprised by said surface. In one embodiment, said hydrophilic block is oriented towards said surface such that a longitudinal axis parallel to the longitudinal extension of said hydrophilic block is about perpendicular to a tangent line of said surface. In one embodiment, said hydrophilic block is oriented towards said surface such that a longitudinal extension of said hydrophilic block is about parallel to a radius of said nanoparticle. In one embodiment, the block copolymer is an amphiphilic polymer. Typically, an amphiphilic polymer has a hydrophilic moiety and a hydrophobic moiety. In one embodiment, the nanoparticle comprises a hydrophobic core and a hydrophilic shell, wherein said hydrophobic core comprises said hydrophobic block of said block copolymer and said hydrophilic shell comprises said hydrophilic block of said block copolymer.

In one embodiment, the term "hydrophobic block", as used herein, relates to a block of a block copolymer comprising or consisting of a hydrophobic polymer. In one embodiment, the hydrophobic block comprises or consists of a polymer selected from poly(lactic-co-glycolic acid), polylactic acid, polyglycolic acid, polystyrene, polypropylene sulfide, poly($\varepsilon$-caprolactone), and any combination thereof. In one embodiment, the hydrophobic polymer is selected from poly(lactic-co-glycolic acid), polylactic acid, polyglycolic acid, polystyrene, polypropylene sulfide, poly($\varepsilon$-caprolactone), and any combination thereof; preferably poly(lactic-co-glycolic acid). In a preferred embodiment, the hydrophobic block comprises or consists of poly(lactic-co-glycolic acid). In one embodiment, the hydrophobic block is a PLGA block. In one embodiment, the hydrophobic block is PLGA.

In one embodiment, the term "hydrophilic block", as used herein, relates to a block of a block copolymer comprising or consisting of a hydrophilic polymer, e.g. a hydrophilic polymer selected from alkoxy polyethylene glycols, polyacrylic acid, polyethylenimine, and any combination thereof. In one embodiment, the hydrophilic block comprises or consists of a polymer selected from alkoxy polyethylene glycols, preferably selected from $C_1$-$C_4$ alkoxy polyethylene glycols, more preferably methoxy polyethylene glycols. In one embodiment, the hydrophilic block comprises or consists of methoxy polyethylene glycol, ethoxy polyethylene glycol, propoxy polyethylene glycol, and/or butoxy polyethylene glycol, preferably comprises or consists of methoxy polyethylene glycol. In a preferred embodiment, the hydrophilic block comprises or consists of methoxy polyethylene glycol. In one embodiment, the terms "methoxy polyethylene glycol" and "$C_1$ alkoxy polyethylene glycol" are used interchangeably. The inventors have found that the nanoparticle exhibits a particularly advantageous relation between hydrophilicity and hydrophobicity with alkoxy polyethylene glycols selected from $C_1$-$C_4$ alkoxy polyethylene glycols. Particularly with respect to the preparation of the nanoparticle and the arrangement of the block copolymers, the inventors have found that it is advantageous to provide the nanoparticle with an alkoxy polyethylene glycol selected from $C_1$-$C_4$ alkoxy polyethylene glycols. The nanoparticle comprising alkoxy polyethylene glycol has the advantage that the alkoxy polyethylene glycol reduces the interaction of the nanoparticles with the interstitium at the injection site, thus facilitating an efficient transport towards the sentinel lymph node. Furthermore, the occurrence of pain at the injection site is reduced, since the interaction of the nanoparticle with the injection site is reduced by alkoxy polyethylene glycol. In one embodiment, the hydrophilic block is an alkoxy polyethylene glycol block, preferably a methoxy polyethylene glycol block. In one embodiment, the hydrophilic block is alkoxy polyethylene glycol, preferably methoxy polyethylene glycol.

The term "alkoxy" means a group having the formula -O-alkyl, in which an alkyl group is attached to the parent molecule, e.g. polyethylene glycol, via an oxygen atom. The alkyl portion of an alkoxy group can have 1 to 20 carbon atoms (i.e., $C_1$-$C_{20}$ alkoxy), 1 to 12 carbon atoms (i.e., $C_1$-$C_{12}$ alkoxy), or 1 to 4 carbon atoms (i.e., $C_1$-$C_4$ alkoxy). Examples of suitable alkoxy groups include, but are not limited to, methoxy (-O-$CH_3$ or OMe), ethoxy (-$OCH_2CH_3$ or -OEt), t-butoxy (-O-$C(CH_3)_3$ or -OtBu) and the like. Such alkoxy group may itself be substituted once or several times, e.g. with a substituent selected from halogen, -NH2, -COOH, -OH, -SH, - maleimide, and any combination thereof. The term "alkyl" refers to a monovalent straight or branched chain, saturated aliphatic hydrocarbon radical having a number of carbon atoms any of which is optionally substituted. In one embodiment, the alkoxy polyethylene glycol, preferably methoxy polyethylene glycol, has a molecular weight in the range of from 200 to 20000 Da, preferably in the range of from 1000 to 10000 Da, more preferably in the range of from 2000 to 8000 Da, e.g. about 5000 Da.

In one embodiment, the nanoparticle, the block copolymer, and/or the hydrophilic block comprises, optionally in addition to said alkoxy polyethylene glycol, polyethylene glycol or a functionalized polyethylene glycol derivative, preferably functionalized with -NH2 or -COOH. For example, the nanoparticle may comprise a functionalized polyethylene glycol derivative, e.g. functionalized with -NH2 or -COOH, for coupling the targeting agent to the nanoparticle. In one embodiment, the nanoparticle, the block copolymer, and/or the hydrophilic block comprises a charged moiety, e.g. a negatively charged moiety and/or a positively charged moiety. In one embodiment, the nanoparticle has a $\varsigma$-potential that is positive, negative, or neutral, for example of from -20 mV to 0 mV, or of from -15 mV to -5 mV.

In one embodiment, said block copolymer is a diblock copolymer or triblock copolymer, preferably a diblock copolymer, more preferably a methoxy-poly(ethylene glycol)-block-poly(lactide-co-glycolide). Block copolymers typically comprise two or more homopolymer subunits, e.g. an alkoxy polyethylene glycol subunit and a poly(lactic-co-glycolic acid) subunit, linked by covalent bonds. Diblock copolymers have two distinct blocks, e.g. an alkoxy polyethylene glycol block and a poly(lactic-co-glycolic acid) block; triblock copolymers have three blocks, e.g. a first alkoxy polyethylene glycol block, a poly(lactic-co-glycolic acid) block, and a second alkoxy polyethylene glycol block. For example, a block may be a portion of a macromolecule, comprising many units, that has at least one feature which is not present in the adjacent portions. A possible sequence of repeat units A and B in a diblock copolymer and in a triblock copolymer might be, for example, ~A-A-A-A-A-A-A-B-B-B-B-B-B-B~ and ~A-A-A-A-A-A-A-B-B-B-B-B-B-A-A-A-A-A~, respectively. In one embodiment, the block copolymer is an alkoxy-poly(ethylene glycol)-block-poly(lactide-co-glycolide) such as a $C_1$-$C_4$ alkoxy-poly(ethylene glycol)-block-poly(lactide-co-glycolide), preferably methoxy-poly(ethylene glycol)-block-poly(lactide-co-glycolide).

In one embodiment, the terms "methoxy-poly(ethylene glycol)-block-poly(lactide-co-glycolide)", "MeO-PEG-PLGA", "methoxy-terminated poly(ethylene glycol)-poly(lactic-co-glycolic acid)", "methoxy poly(ethylene glycol)-poly(lactic-co-glycolic acid)", and "a-Methyl-ω-hydro-poly(ethylene oxide)-block-poly(lactide-co-glycolide)" are used interchangeably. In one embodiment, the methoxy polyethylene glycol block of said MeO-PEG-PLGA has a molecular weight in the range of from 200 to 20000 Da, preferably in the range of from 1000 to 10000 Da, more preferably in the range of from 2000 to 8000 Da, e.g. about 5000 Da. In one embodiment, the PLGA block of said MeO-PEG-PLGA has a molecular weight in the range of from 1000 to 30000 Da, preferably in the range of from 4000 to 20000 Da, more preferably in the range of from 8000 to 15000 Da, e.g. about 12000 to about 13000 Da. In one embodiment, the block copolymer is MeO-$PEG_{5k}$-$PLGA_{13k}$. Advantageously, MeO-PEG-PLGA is biodegradable and biocompatible, which facilitates clinical applicability e.g. compared to labelled albumin particles which are blood products.

In one embodiment, said core of the nanoparticle further comprises a polymer different from said block copolymer; said polymer optionally being selected from poly(lactic-co-glycolic acid), polylactic acid, polyglycolic acid, polystyrene, polypropylene sulfide, poly(ε-caprolactone), and any combination thereof; said polymer preferably comprising or consisting of poly(lactic-co-glycolic acid). For example, the core of the nanoparticle may comprise the block copolymer or a part of the block copolymer, e.g. the hydrophobic block of the block copolymer such as a poly(lactic-co-glycolic acid) subunit of methoxy-poly(ethylene glycol)-block-poly(lactide-co-glycolide), and may further comprise the polymer different from said block copolymer, e.g. poly(lactic-co-glycolic acid).

In one embodiment, said nanoparticle has a size in a range of from 5 nm to 200 nm, preferably in a range of from 10 nm to 60 nm, more preferably in a range of from 10 nm to 50 nm, even more preferably in a range of from 13.5 nm to 30 nm. In one embodiment, the nanoparticle size is measured using dynamic light scattering, particle scattering diffusometry, nanoparticle tracking analysis, atomic force microscopy, or transmission electron microscopy, preferably dynamic light scattering. The size of a particle is determined by its diameter which relates to any straight line segment that passes through the center of a spherical particle and whose endpoints lie on the spherical particle. In cases of non-spherical particles, a diameter relates to the longest line segment that passes through the center of said non-spherical particle and whose endpoints lie on the particle. In one embodiment, a mean diameter relates to the mean of the diameters of nanoparticles comprised in a batch of nanoparticles. In one embodiment, the particles of the present invention have a polydispersity index of from 0.01 to 0.5, preferably from 0.01 to 0.3, more preferably from 0.01 to 0.1. In one embodiment, the polydispersity index is measured using dynamic light scattering, particle scattering diffusometry, nanoparticle tracking analysis, atomic force microscopy, or transmission electron microscopy, preferably dynamic light scattering. In one embodiment, the hydrodynamic diameter and/or the polydispersity index is determined by dynamic light scattering (DLS), preferably using a Malvern ZetaSizer Nano ZS (e.g. Malvern Instruments GmbH, Lappersdorf, Germany) with a 633 nm He-Ne laser (e.g. 173° backscatter angle), e.g. at 25 °C. In one embodiment, the nanoparticle has a hydrodynamic diameter in the range of from about 10 nm to about 100 nm, preferably of from about 25 nm to about 50 nm. In one embodiment, said nanoparticles have a size in a range of from 10 nm to 60 nm with at least 85% of said nanoparticles having a size < 30 nm, preferably in a range of from 10 nm to 50 nm with at least 85% of said nanoparticles having a size < 30 nm. In one embodiment, said nanoparticle is sterile filtered and/or sterile filterable, e.g. with a filter having a pore size of about 0.2 μm.

In one embodiment, said nanoparticle comprises a label, optionally a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{124}$I, $^{125}$I, $^{131}$I, $^{169}$Er, $^{177}$Lu, $^{186}$Re, $^{188}$Re, $^{198}$Au, and any combination thereof; even more preferably [99m]Tc. In one embodiment, the terms "label" and "marker" are used interchangeably. In one embodiment, the label is configured such that it allows to be detected in any imaging technique known to the skilled person, e.g. detected using SPECT, PET, CT, MRT, and/or fluorescence imaging. In one embodiment, the radioactive label is selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{124}$I, $^{125}$I, $^{131}$I, $^{169}$Er, $^{177}$Lu, $^{186}$Re, $^{188}$Re, $^{198}$Au, and any combination thereof. The radiolabeled nanoparticles used for treatments are quite similar to those used for diagnostic purposes and differ mainly in the type of radionuclides used for labeling, e.g., beta emitters such as $^{90}$Y, $^{169}$Er, $^{177}$Lu, or $^{186}$Re. In one embodiment, the fluorescent label is selected from fluorescent dyes, particularly pharmaceutically acceptable fluorescent dyes; preferably is selected from cyanine dyes, porphyrins, and combinations thereof; more preferably is selected from indocyanine green and protoporphyrin IX. For example, a fluorescent label may be selected from xanthene derivatives, such as fluorescein, rhodamine, Oregon green, and eosin; cyanine derivatives, such as cyanine, indocarbocyanine, oxacarbocyanine, thiacarbocyanine, merocyanine, indocyanine green, and infracyanine green; squaraine derivatives or ring-substituted squaraines, including Seta, SeTau, and Square dyes; naphthalene derivatives such as dansyl and prodan derivatives; coumarin derivatives; oxadiazole derivatives, such as pyridyloxazole, nitrobenzoxadiazole, and benzoxadiazole; anthracene derivatives, such as anthraquinones, including DRAQ5, DRAQ7, and CyTRAK Orange; pyrene derivatives, such as cascade blue; oxazine derivatives, such as Nile red, Nile blue, cresyl violet, oxazine 170; acridine derivatives, such as proflavin, acridine orange, acridine yellow; arylmethine derivatives, such as auramine, crystal violet, malachite green; and tetrapyrrole derivatives, such as porphin, phthalocyanine, bilirubin. For example, a fluorescent dye may be selected from Alexa Fluor dyes; coumarin dyes, including 3-cyano-7-hydroxycoumarin, 6,8-difluoro-7-hydroxy-4-methylcoumarin, 7-amino-4-methylcoumarin, 7-ethoxy-4-trifluoromethylcoumarin, 7-hydroxy-4-methylcoumarin, 7-hydroxycoumarin-3-carboxylic acid, 7-dimethylamino-coumarin-4-acetic acid, 7-amino-4-methyl-coumarin-3-acetic acid, and 7-diethylamino-coumarin-3-carboxylic acid; BODIPY® dyes; Quantum Dots; Oregon Green® dyes; LanthaScreen® Tb Chelates; Rhodamine 110, Rhodamine Green, Rhodamine Red, Texas Red-X, Cascade Blue, Pacific Blue, Marina Blue, Pacific Orange, Dapoxyl Sulfonyl Chloride, Dapoxyl Carboxylic Acid, 1-Pyrenebutanoic Acid, 1-Pyrenesulfonyl Chloride, 2-(2,3-Naphthalimino) ethyl Trifluoromethanesulfonate, 2-Dimethylaminonaphthalene-5-Sulfonyl Chloride, 2-Dimethylaminonaphthalene-6-Sulfonyl Chloride, 3-Amino-3-Deoxydigoxigenin Hemisuccinamide, 4-Sulfo-2,3,5,6-Tetrafluorophenol, 5-(and-6)-Carboxyfluorescein, 5-(and-6)-Carboxynaphthofluorescein, 5-(and-6)-Carboxyrhodamine 6G, 5-(and-6)-Carboxytetramethylrhodamine, 5-(and-6)-Carboxy-X-Rhodamine, 5-Dimethylaminonaphthalene-1-Sulfonyl Chloride (Dansyl Chloride), 6-((5-Dimethylaminonaphthalene-1-Sulfonyl)amino) Hexanoic Acid, Succinimidyl Ester (Dansyl-X, SE), Lissamine Rhodamine B Sulfonyl Chloride, Malachite Green isothiocyanate, NBD Chloride, 4-Chloro-7-Nitrobenz-2-Oxa-1,3-Diazole (4-Chloro-7-Nitrobenzofurazan), NBD Fluoride; 4-Fluoro-7-Nitrobenzofurazan, 4-Fluoro-7-Nitrobenz-2-Oxa-1,3-Diazole, NBD-X, and PyMPO; CyDyes®; ATTO dyes; DY dyes; CF™ dyes; CAL Fluor® dyes; Quasar® dyes; Biosearch Blue and Pulsar® 650; DyLight® Fluor dyes; FluoProbes® dyes; SeTau dyes; Square dyes; Seta dyes; SQ-565 and SQ-780; Chromeo™ dyes; Abberior STAR dyes; Abberior CAGE dyes; Abberior FLIP 565; IRDye® Infrared Dye; Tide Fluor™ dyes; iFluor™ dyes; mFluor™ dyes; trFluor™ Eu and trFluor Tb; HiLyte Fluor™ dyes; Terbium Cryptate and Europium Cryptate; and other nucleotide classical dyes, including FAM, TET, JOE, VIC™, HEX, NED, TAMRA, ROX™, Texas Red®.

In one embodiment, the radioluminescent label is selected from $^{131}$I, $^{124}$I, $^{89}$Zr, $^{68}$Ga, $^{64}$Cu, $^{44}$Sc, and $^{18}$F. In one embodiment, the dye is a pharmaceutically acceptable dye. In one embodiment, the dye is visible to the human eye. For example, the dye, e.g. the pharmaceutically acceptable dye, may be selected from methylene blue, isosulfan blue, acid fuchsine, acid red, alba red, alizarin cyanine green, alizarin violet, alizurol purple ss, allura red ac, allura red ac, alphazurine fg, amaranth, brilliant black bn, brilliant blue FCF, brown HT, carmoisine, copper phthalocyanine, dibromofluorescein, diiodofluorescein, eosine, erythrosine, fast green FCF, flaming red, fluorescein, green s, helindone pink cn, indigo, indigotine, lithol rubin B, lithol rubin B CA, napthol yellow S, orange II, patent blue V, phloxine B, ponceau 4R, ponceau SX, pyranine concentrated, quinizarine green ss, quinoline yellow, resorcin brown, rose bengal, sunset yellow FCF, tartrazine, tetrabromo fluorescein, tetrachlorotetra-bromofluorescein, toney red, uranine, and any combination thereof. In one embodiment, the dye is selected from methylene blue, isosulfan blue, patent blue V, and Brilliant blue FCF, e.g. selected from methylene blue and isosulfan blue. In one embodiment, the nanoparticle comprises at least one label, optionally two or more labels, preferably comprises a radioactive label and a further label selected from a fluorescent label, a radioluminescent label, a magnetic label, and a dye. In one embodiment, the nanoparticle comprises a radioactive label and a fluorescent label, e.g. selected from fluorescent dyes such as indocyanine green and protoporphyrin IX. In one embodiment, the nanoparticle comprises a radioactive label and a radioluminescent label. In one embodiment, the nanoparticle comprises a radioactive label and a dye, e.g. selected from methylene blue and isosulfan blue. The inventors have found that the nanoparticle comprising a label, particularly a radioactive label, allows to localize regions of interest, e.g. metastases and/or lymph nodes, with high precision. Furthermore, the inventors have found that the precision of a localization of a region of interest, e.g. the localization of a metastasis and/or sentinel lymph node, can be even further enhanced with a nanoparticle which comprises a further label such as a fluorescent label, a radioluminescent label, a magnetic label, and/or a dye, in addition to a radioactive label. Moreover, an advantage of a nanoparticle comprising a radioactive label and a further label, such as a fluorescent dye or a dye, is that the precision of detection is enhanced, since regions of interest on the surface of the surgical area can be observed using the further label, such as a fluorescent dye or a dye (e.g. observed with the naked eye in case of a dye; or by means of additional illumination and, if necessary, filter glasses in case of a fluorescent dye) and the regions of interest localized deeper in the tissue can be observed by detecting the signal of the radioactive label. The two or more labels can be configured such that they complement each other. In one embodiment, said label has a radioactivity, preferably at a time of preparation, in a range of from 4 MBq to 8000 MBq, preferably in a range of from 400 MBq to 2200 MBq, more preferably in a range of from 400 MBq to 1000 MBq. In one embodiment, the term "radioactivity at a time of preparation" relates to an initial radioactivity of the label during the labelling of the nanoparticles. Typically, the radioactivity of the label at a time when the nanoparticle is administered to the patient is lower than the initial radioactivity of the label during the labelling of the nanoparticles. For example, the radioactivity of the label at a time when the nanoparticle is administered to the patient may be ≤ 10 MBq. In one embodiment, the label associates with said nanoparticle, preferably binds to said nanoparticle, by interacting with the hydrophilic block, the hydrophobic block, or both. For example, a label such as $^{99m}$Tc may bind to the hydrophobic block such as PLGA of the block copolymer. In one embodiment, the nanoparticle of the invention and/or the composition of the invention has/have a radiochemical purity of at least 95%, preferably of at least 99%. In one embodiment, radiochemical purity is measured by thin layer chromatography (TLC). In one embodiment, the nanoparticle of the invention is a detection agent and/or radiotracer. In one embodiment, the nanoparticle is a labelled nanoparticle for medical imaging. In one embodiment, the nanoparticle is a labelled MeO-PEG-PLGA nanoparticle for sentinel lymph node imaging. In one embodiment, the nanoparticle is a $^{99m}$Tc-labelled MeO-PEG-PLGA nanoparticle for sentinel lymph node imaging. In one embodiment, the magnetic label is selected from paramagnetic labels and superparamagnetic labels, preferably is selected from gadolinium and iron oxide. In one embodiment, a nanoparticle comprising a magnetic label is for use in magnetic resonance imaging (MR/MRT).

In one embodiment, said nanoparticle comprises a targeting agent, preferably a lymph node targeting agent and/or a tumor targeting agent; wherein, preferably, said targeting agent comprises or consists of a ligand, a moiety-binding motif, an enzyme inhibitor, and/or an antigen-binding peptide; wherein, more preferably, said targeting agent comprises or consists of a ligand, an enzyme inhibitor, and/or an antigen-binding peptide. A nanoparticle comprising a targeting agent is advantageous for medical imaging, for example, a targeting agent allows to increase the retention of the nanoparticle in the sentinel lymph node. In one embodiment, the ligand is selected from agonistic receptor ligands and antagonistic receptor ligands, e.g. somatostatin receptor ligands, neurotensin receptor ligands, neuropeptide Y receptor ligands, and dopamine receptor ligands; and binding motifs, e.g. lysine-urea-glutamate for prostate-specific membrane antigen, arginyl-glycyl-aspartic acid for integrin receptors, and mannose for mannose receptors. In one embodiment, the moiety-binding motif binds to a moiety such as a peptide on a target, e.g. on a target such as a protein, a cancer cell,or lymph node. In one embodiment, the moiety-binding motif binds to a protein on a cell surface. In one embodiment, the moiety-binding motif is selected from antibodies and antigen-binding fragments thereof, aptamers, sugars, peptides, and proteins, preferably is selected from lysine-urea-glutamate, arginyl-glycyl-aspartic acid, and mannose. In one embodiment, the term "targeting agent" relates to a moiety capable of binding a target on a cell surface, preferably specifically binding said target, e.g. an antibody or an antigen-binding peptide thereof, for example an IgG, a Fab fragment, a single-chain Fab fragment, or a single domain antibody, a DNA and/or RNA aptamer, a peptide, a binding protein, a ligand of a cell-surface receptor, or a lipid. The term "antigen-binding peptide", as used herein, relates to a peptide which specifically binds to an antigen, e.g. an antigen on a cancer cell and/or a cell of the lymph system. In one embodiment, an antigen-binding peptide is based on an immunoglobulin, such as a polyclonal or monoclonal antibody, or based on a protein scaffold structure having antigen-binding capacity, such as an anticalin protein, an Affilin, an Affimer, an Affitin, an Alphabody, a nanobody, or a DARPin. Advantageously, the targeting agents increase the uptake and retention of the nanoparticle in the sentinel lymph node or at target cells. For example, targeting agents binding to surface receptors of macrophages and dendritic cells increase the retention time in the lymph node or at target cells.

The present invention also relates to a composition, preferably a pharmaceutical composition, comprising a nanoparticle, as defined herein, and a pharmaceutically or agriculturally acceptable excipient. In one embodiment, said composition is for use in medicine. In a further embodiment, the nanoparticle of the invention and/or the composition of the invention is used for applying the nanoparticle to a plant, e.g. for imaging molecular mechanisms of the plant such as transport or deposition of nanoparticles within or on the plant.

The present invention further relates to a nanoparticle, as defined herein, or pharmaceutical composition, as defined herein, for use in medicine, e.g. for use as a medicament. The present invention further relates to a nanoparticle, as defined herein, or pharmaceutical composition, as defined herein, for use in a method of preventing, treating, monitoring, and/or diagnosing a disease, optionally a disease selected from malignant diseases, diseases associated with the lymphatic system, a disease in a preformed body cavity, and joint diseases. In one embodiment, said method, e.g. said monitoring and/or diagnosing a disease, comprises localizing said disease, particularly localizing said disease using said nanoparticle.

In one embodiment, said nanoparticle and/or said pharmaceutical composition are for use in a method of preventing, treating, monitoring, and/or diagnosing a disease, optionally selected from a malignant disease, a disease associated with the lymphatic system, a disease in a preformed body cavity, and a joint disease, comprising administering the nanoparticle and/or the pharmaceutical composition to a patient in need thereof. In one embodiment, said administering comprises administering an effective amount of a nanoparticle, as defined herein, and/or a pharmaceutical composition, as defined herein, to a patient in need thereof. In one embodiment, said nanoparticle or said pharmaceutical composition are used for the manufacture of a medicament, preferably a medicament for preventing, treating, monitoring, and/or diagnosing a disease.

In one embodiment, the term "disease" relates to any disease known to the person skilled in the art, and comprises any manifestation of a disease. For example, the disease may be a malignant disease, a disease associated with the lymphatic system, a disease in a preformed body cavity, a joint disease, and/or an inflammatory disease. In one embodiment, the malignant disease is a disease in which abnormal cells divide without control and optionally invade nearby tissues. In one embodiment, the malignant disease is cancer. In one embodiment, a disease associated with the lymphatic system is any disease in which the lymphatic system is involved and/or any disease which affects the lymphatic system, e.g. a lymphatic disease or a disease present within the lymphatic system such as a metastasis present within a lymph node. In one embodiment, the disease associated with the lymphatic system is any disease which directly or indirectly affects the lymphatic system. In one embodiment, the disease associated with the lymphatic system is a lymphatic disease. In one embodiment, the malignant disease is selected from breast cancer, malignant melanoma, skin cancer, vulvar carcinoma, cervical carcinoma, penile carcinoma, prostate cancer, and head and neck squamous cell carcinoma. In one embodiment, the disease associated with the lymphatic system is selected from lymphedema, lymphocele, lymphangitis, and congenital, iatrogenic, immunogenic, autoimmunogenic, injury-related, postinfectious, postinflammatory, posttherapeutic, posttraumatic, radiation-induced or secondary to other conditions induced alterations of the lymphatic system, preferably is selected from lymphedema, lymphocele, and lymphangitis.

The present invention further relates to a nanoparticle, as defined herein, or pharmaceutical composition, as defined herein, for use in medical imaging, radioguided surgery, and/or radionuclide therapy, preferably in scintigraphy such as lymphoscintigraphy, single-photon emission computed tomography (SPECT), positron emission tomography (PET), and/or in radiosynovectomy. In one embodiment, the nanoparticle and/or pharmaceutical composition are for use in medical imaging of lymph nodes, preferably for medical imaging of the sentinel lymph node. In one embodiment, the nanoparticle and/or pharmaceutical composition are for use in imaging and intraoperative detection of a sentinel lymph node, e.g. for patients having any of breast cancer, malignant melanoma, skin cancer, vulvar carcinoma, cervical carcinoma, penile carcinoma, prostate cancer, and head and neck cancer. The nanoparticle and/or pharmaceutical composition may also be used for imaging of regional lymphatic flow, e.g. for individualised radiation therapy, and/or for lymphatic flow scintigraphy, e.g. for diagnosing lymphatic oedema in the limbs. For example, the nanoparticle and composition may be used for SPECT or PET, optionally in combination with X-ray computed tomography (e.g. SPECT/CT or PET/CT) or magnetic resonance imaging (e.g. PET/MR). For example, a nanoparticle comprising a label such as $^{99m}$Tc is highly advantageous for SPECT, and nanoparticles comprising a label such as $^{18}$F, $^{44}$Sc, $^{64}$Cu, $^{68}$Ga, and/or $^{89}$Zr are highly advantageous for PET. In one embodiment, said medical imaging, radioguided surgery, and/or radionuclide therapy comprises imaging or irradiating a component of the lymph system, e.g. a lymph node, a metastasis, a tumor, a body cavity, and/or a cyst. For example, the nanoparticle can be used to irradiate and/or to image joint diseases such as degenerative or inflammatory joint diseases, e.g. in radiosynovectomy; tumor diseases, e.g. tumors in a pleural cavity or a cystic brain tumor; lymphatic diseases, e.g. lymphocele; and inflammatory diseases such as inflammations in the paranasal sinuses or in the middle ear, e.g. in radiotympanosinuorthesis. For such medical imaging, such radioguided surgery, and/or such radionuclide therapy, the nanoparticle, preferably a labelled nanoparticle, may be injected and/or instillated into a tissue and/or body fluid and/or preformed body cavity, e.g. injected into the lymph system, into a malignant tissue, into a joint, into a cyst, into a paranasal sinus, or into a lymphocele.

The term "administering", as used herein, relates to the administration of an agent, e.g. a nanoparticle of the present invention and/or a pharmaceutical composition of the present invention, which can be accomplished by any method which allows the agent to reach the target cells. These methods include, for example, injection, instillation, oral ingestion, inhalation, nasal delivery, topical administration, deposition, implantation, suppositories, or any other method of administration where access to the target cells by the nanoparticle is obtained. An injection may relate to an intravenous, intraarterial, intradermal, subcutaneous, subareolar at the breast, periareolar at the breast, intratumoral, peritumoral, intramucosal, submucosal, intraprostatic, intracervical at the uterine cervix, paracervical at the uterine cervix, intramuscular or intraperitoneal injection. Instillation may refer to intraarticular, intrapleural, pericardial, intracystic, intrasinusoidal, paranasal-intrasinusoidal, maxillary-intrasinusoidal, frontal-intrasinusoidal, sphenoidal-intrasinusoidal, ethmoidal-intrasinusoidal or transtympanic instillation. In one embodiment, an "effective amount" is an amount of the nanoparticle or the pharmaceutical composition that evokes a desired effect, e.g. that alleviates symptoms as found for the disease and/or that allows to localize the disease. The term "patient", as used herein, relates to a human or an animal, preferably a mammal. Treating of a patient is meant to include, e.g., preventing, treating, reducing the symptoms of, or curing the disease or condition, for example cancer, inflammations of the paranasal sinuses, joint diseases, or diseases associated with the lymphatic system, e.g. lymphatic diseases.

The present invention also relates to a method of preparing a nanoparticle, preferably a nanoparticle as defined herein, comprising

a) providing a block copolymer comprising or consisting of a hydrophobic block and a hydrophilic block, wherein said hydrophobic block comprises or consists of a hydrophobic polymer, preferably poly(lactic-co-glycolic acid), and wherein said hydrophilic block comprises alkoxy polyethylene glycol, preferably selected from $C_1$-$C_4$ alkoxy polyethylene glycol, more preferably methoxy polyethylene glycol;

b) dissolving said block copolymer in an organic solvent, preferably selected from acetonitrile, N,N-dimethylformamide, acetone, tetrahydrofuran, dimethyl sulfoxide, and any combination thereof, more preferably acetonitrile, thereby obtaining a polymer solution;

c) optionally, adding a polymer different from said block copolymer, said polymer preferably comprising or consisting of poly(lactic-co-glycolic acid), to said polymer solution;

d) contacting, particularly mixing, said polymer solution obtained in step b) or step c) with an aqueous phase, preferably using a microfluidic system;

e) obtaining nanoparticle(s), preferably by self-assembly, more preferably by nanoprecipitation; said nanoparticle(s) optionally having a size in a range of from 5 nm to 200 nm, preferably in a range of from 10 nm to 60 nm, more preferably in a range of from 10 nm to 50 nm, even more preferably in a range of from 13.5 nm to 30 nm;

f) optionally, lyophilizing said nanoparticle(s) to obtain lyophilized nanoparticle(s).

In one embodiment, steps a) - c) are performed in any order, e.g. step c) is performed prior to or after step b). In one embodiment, the block copolymer provided in step a) is a diblock copolymer or triblock copolymer, preferably a diblock copolymer. In one embodiment, the block copolymer provided in step a) is an alkoxy-poly(ethylene glycol)-block-poly(lactide-co-glycolide) such as a $C_1$-$C_4$ alkoxy-poly(ethylene glycol)-block-poly(lactide-co-glycolide), preferably methoxy-poly(ethylene glycol)-block-poly(lactide-co-glycolide).

In one embodiment, the method comprises step c) of adding a polymer different from said block copolymer, said polymer preferably comprising or consisting of poly(lactic-co-glycolic acid), to said polymer solution; wherein, preferably, said block copolymer and said polymer different from said block copolymer have a weight ratio of about 7:3 (m/m). The nanoparticle comprising a polymer different from said block copolymer, e.g. poly(lactic-co-glycolic acid), has the advantage that the stability of the nanoparticle is even further enhanced. The inventors have found that a nanoparticle comprising a polymer different from said block copolymer, e.g. poly(lactic-co-glycolic acid), has very high stability. In one embodiment, a concentration of said block copolymer in said polymer solution obtained in step b) is about 1 mg/ml to about 30 mg/ml, preferably about 10 mg/ml to about 20 mg/ml, e.g. about 20 mg/ml. In one embodiment, a concentration of said block copolymer and a concentration of said polymer different from said block copolymer in said polymer solution obtained in step c) each is about 1 mg/ml to about 30 mg/ml, preferably about 10 mg/ml to about 20 mg/ml, e.g. about 20 mg/ml. In one embodiment, the block copolymer of said polymer solution obtained in c), e.g. methoxy-poly(ethylene glycol)-block-poly(lactide-co-glycolide), has a molar concentration of about $1 \times 10^{-4}$ mol/l to about $15 \times 10^{-4}$ mol/l, e.g. about $7.85 \times 10^{-4}$ mol/l. In one embodiment, the polymer different from said block copolymer of said polymer solution obtained in c), e.g. poly(lactic-co-glycolic acid), has a molar concentration of about $1 \times 10^{-4}$ mol/l to about $12 \times 10^{-4}$ mol/l, e.g. about $5.45 \times 10^{-4}$ mol/l.

In one embodiment, said contacting, particularly mixing, said polymer solution with an aqueous phase in step d) is performed by microfluidics. In one embodiment, said contacting, particularly mixing, said polymer solution with an aqueous phase in step d) is performed using a microfluidic system. Advantageously, a contacting, particularly mixing, using microfluidics allows for an efficient production of stable nanoparticles, particularly nanoparticles with a defined size, e.g. a size in a range of from 10 nm to 60 nm such as in a range of from 10 nm to 50 nm or in a range of from 13.5 nm to 30 nm. Particles between 10 and 50 nm in size are ideal for medical imaging, particularly sentinel lymph node imaging, as they rapidly leave the injection site and are taken up into the lymphatic system and then are strongly retained in the first lymph nodes. Particles larger than 50 nm may remain at the injection site while smaller radiotracers (< 10 nm) may continue to flow to distal lymph nodes. In one embodiment, the microfluidic system is a microfluidic device.

In one embodiment, said contacting, particularly mixing, comprises providing a laminar flow, particularly a laminar flow of said polymer solution and said aqueous phase. In one embodiment, said contacting, particularly mixing, of said polymer solution with an aqueous phase comprises providing a total flow rate, e.g. within a microfluidic system, of about 1 ml/min to about 350 ml/min, preferably about 4 ml/min to about 30 ml/min, e.g. about 8 ml/min to about 16 ml/min. In one embodiment, said contacting, particularly mixing, of said polymer solution with an aqueous phase comprises providing a flow rate ratio of said aqueous phase to said polymer solution, e.g. within a microfluidic system, of about 1:1 to about 20:1, preferably of about 1:1 to about 10:1, e.g. about 7:1. In one embodiment, said contacting, particularly mixing, of said polymer solution with an aqueous phase comprises providing a flow rate ratio of said aqueous phase to said polymer solution, e.g. within a microfluidic system, of about 1:1 to about 20:1, preferably of about 1:1 to about 10:1, e.g. about 7:1; a total flow rate of about 1 ml/min to about 350 ml/min, preferably about 4 ml/min to about 30 ml/min, e.g. about 8 ml/min to about 16 ml/min; and a concentration of said block copolymer and/or a concentration of said polymer different from said block copolymer in said polymer solution obtained in step c) of about 1 mg/ml to about 30 mg/ml, preferably about 10 mg/ml to about 20 mg/ml, e.g. about 20 mg/ml.

In one embodiment, in step d) said polymer solution obtained in step b) or step c) is contacted, particularly mixed, with said aqueous phase by microfluidic mixing. Advantageously, production of the nanoparticles using microfluidics enables a controlled synthesis of the polymeric particles with a defined size and a narrow size distribution. Microfluidic systems typically contain cartridges with microchannels. Due to their small dimensions, the injected polymer solution and the aqueous phase (antisolvent) are mixed very fast through diffusion resulting in supersaturation of the polymers, which in turn leads to nucleation and particle growth. By using a microfluidic system, particularly a microfluidic device, for particle preparation, critical process steps such as mixing and supersaturation can be influenced by changing the total flow rate, the aqueous to organic solvent flow rate ratio, and the polymer concentration, thereby enabling precise control of NP size and polydispersity index (PDI). Additionally, microfluidics offers a high batch-to-batch reproducibility of the nanoparticles and the possibility to easily scale-up the preparation which is advantageous for later clinical use. Thus, when preparing the carrier particles with a microfluidic system e.g. microfluidic device, nanoparticles with a low PDI can be produced, thereby overcoming the poor size distribution of [$^{99m}$Tc]Tc-sulfur colloids. In one embodiment, said obtaining in step e) comprises obtaining nanoparticles having a polydispersity index of from 0.01 to 0.5, preferably from 0.01 to 0.3, more preferably from 0.01 to 0.1; said polydispersity index optionally being determined by dynamic light scattering (DLS), preferably using a Malvern ZetaSizer Nano ZS (e.g. Malvern Instruments GmbH, Lappersdorf, Germany) with a 633 nm He-Ne laser (e.g. 173° backscatter angle), e.g. at 25 °C.

In one embodiment, said method further comprises labelling, preferably radioactively labelling, said nanoparticle(s) obtained in step e) or said lyophilized nanoparticle(s) obtained in step f); said labelling preferably comprising

i) providing a label, optionally a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{125}$I, $^{125}$I, $^{131}$I, $^{169}$Er, $^{177}$Lu, $^{186}$Re, $^{188}$Re, $^{198}$Au, and any combination thereof; even more preferably $^{99m}$Tc, preferably $^{99m}$Tc pertechnetate, more preferably a $^{99m}$Tc pertechnetate solution; and mixing said label, preferably $^{99m}$Tc pertechnetate, with said nanoparticle(s) obtained in step e) and/or said lyophilized nanoparticle(s) obtained in step f);
ii) optionally, adding a reducing agent, preferably SnCl$_2$, thereby obtaining a reduced label, preferably reduced $^{99m}$Tc pertechnetate;
iii) optionally, adding a stabilizer, preferably ascorbic acid and/or ethanol;
iv) allowing said label, optionally said reduced label obtained in step ii), preferably reduced $^{99m}$Tc pertechnetate obtained in step ii), to label said nanoparticle(s);
v) obtaining labelled nanoparticle(s).

The inventors have found that a high labelling efficiency can be achieved with a method comprising steps i), iv), and v), and optionally steps ii) and/or iii). Particularly, the inventors have found that a method comprising steps i)-v) achieves a highly advantageous labelling efficiency. The nanoparticles labelled with the above mentioned method steps allow to achieve a good signal intensity for medical imaging, e.g. sentinel lymph node imaging, and/or radioguided surgery.

In one embodiment, step i) comprises providing more than one label, e.g. two or more labels, preferably comprises providing a radioactive label and a further label selected from a fluorescent label, a radioluminescent label, a magnetic label, and a dye. In one embodiment, step i) comprises providing a radioactive label and a fluorescent label, e.g. selected from fluorescent dyes such as indocyanine green and protoporphyrin IX. In one embodiment, step i) comprises providing a radioactive label and a radioluminescent label. In one embodiment, step i) comprises providing a radioactive label and a dye, e.g. selected from methylene blue and isosulfan blue. In one embodiment, said mixing in step i) comprises mixing said label, preferably $^{99m}$Tc pertechnetate, with said nanoparticle(s) at a molar ratio of about 1:1. In one embodiment, said mixing is performed using a microfluidic system. In one embodiment, said label, preferably $^{99m}$Tc pertechnetate, has a radioactivity, preferably at a time of preparation, in a range of from 4 MBq to 8000 MBq, preferably in a range of from 400 MBq to 2200 MBq, more preferably in a range of from 400 MBq to 1000 MBq.

In one embodiment, said adding in step ii) comprises providing a molar ratio of said reducing agent, preferably SnCl$_2$, to said label, preferably $^{99m}$Tc pertechnetate, in a range of from about 1000:1 to about 100000:1, optionally in a range of from about 3000:1 to about 30000:1. In one embodiment, the label is $^{99m}$Tc and the method comprises step ii) of adding a reducing agent. Depending on the label, the skilled person may or may not perform step ii) of adding a reducing agent. In one embodiment, said reducing agent is SnCl$_2$ or NaBH$_4$. In one embodiment, said reducing agent, e.g. SnCl$_2$, is provided as a solution, e.g. as a solution in HCl. Advantageously, e.g. if the label is $^{99m}$Tc, the formation of unwanted radiocolloids as by-product can be prevented by adding a reducing agent, preferably SnCl$_2$, e.g. at a molar ratio of said reducing agent to said label in a range of from about 1000:1 to about 100000:1.

In one embodiment, said stabilizer in step iii) is selected from ascorbic acid, ethanol, and CoCl$_2$. In one embodiment, if step ii) of adding a reducing agent is present, said stabilizer is added prior to or after adding a reducing agent. Advantageously, an addition a stabilizer such as ascorbic acid stabilizes the product and avoids the formation of radiolysis products.

In one embodiment, said labelling is performed in the absence of oxygen. In one embodiment, steps i), ii), iii), and/or iv) is/are performed in the absence of oxygen. In one embodiment, said labelling, particularly step iv), is performed at a pH of about pH 4.5 to about pH 7.5, preferably at a pH of about pH 4.5 to about pH 5.5, more preferably at about pH 5; and/or for about 1 min to about 6 hours, preferably about 10 min to about 1.5 hours, more preferably about 10 min to about 20 min; and/or at a temperature of about 15°C to about 40°C, preferably at room temperature. Advantageously, the formation of unwanted radiocolloids as by-product can be prevented when performing the labelling at a pH of about pH 4.5 to about pH 7.5, preferably at a pH of about pH 4.5 to about pH 5.5, more preferably at about pH 5. The inventors have found that, advantageously, after completion of the labelling, no free sodium pertechnetate was detectable in the product by thin-layer chromatography. Surprisingly, the method of the invention allows to provide nanoparticles with about 0% impurities (free $^{99m}$Tc pertechnetate, $^{99m}$Tc(IV)-Sn colloids and $^{99m}$Tc(IV) oxide colloids) and with about >99 % radiochemical purity.

In one embodiment, said method comprises coupling a targeting agent, preferably a lymph node targeting agent and/or a tumor targeting agent, to said nanoparticle, preferably by coupling said targeting agent to said block copolymer, optionally by coupling said targeting agent to said block copolymer using ethyl(dimethylaminopropyl) carbodiimide and/or N-hydroxysuccinimide. For example, the targeting agent is a targeting agent as defined above. In one embodiment, said coupling comprises coupling said targeting agent to said nanoparticle using a functionalized polyethylene glycol derivative, e.g. functionalized with -NH2 or -COOH. In one embodiment, said coupling comprises coupling said targeting agent to said nanoparticle using a DCC/NHS-coupling or EDC/NHS-coupling. In one embodiment, the method of the invention comprises sterile filtering the obtained nanoparticle and/or the obtained labelled nanoparticle. Advantageously, the method of the invention allows for an efficient production of nanoparticles including labelled nanoparticles, particularly with a size distribution suitable for medical imaging such as sentinel lymph node imaging.

For the labelling of the nanoparticles of the invention, particularly MeO-PEG-PLGA nanoparticles, the inventors have found that the following parameters/conditions are optimal to obtain stable nanoparticles with a narrow size distribution:

    1. pH 5.
    2. 0.5 mg (=2*10$^{13}$) MeO-PEG-PLGA nanoparticles.
    3. 200 $\mu$g Sn(II) chloride (degassed)
    4. ratio of MeO-PEG-PLGA nanoparticles : $^{99m}$Tc pertechnetate = 1 : 1
    5. order of addition (with exclusion of oxygen):
    providing MeO-PEG-PLGA nanoparticles $\rightarrow$ adding $^{99m}$Tc pertechnetate $\rightarrow$ starting reaction by adding 200 $\mu$g Sn(II) chloride.

The present invention further relates to a kit comprising, preferably in separate containers,

- a nanoparticle as defined herein, optionally in a lyophilized form; a pharmaceutical composition as defined herein; and/or a nanoparticle prepared by a method as defined herein, optionally in a lyophilized form;
- optionally, a label, such as a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{125}$I, $^{125}$I, $^{131}$I, $^{169}$Er, $^{177}$Lu, $^{186}$Re, $^{188}$Re, $^{198}$Au, and any combination thereof; even more preferably $^{99m}$Tc, preferably $^{99m}$Tc pertechnetate, optionally a $^{99m}$Tc pertechnetate solution, e.g. a solution of sodium pertechnetate;
- optionally, a reducing agent, preferably SnCl$_2$; and
- optionally, a bulking agent, a buffer, a cryoprotectant, a stabilizing agent, and/or a tonicity regulating agent.

The kit can be used to provide a labelled nanoparticle of the invention. Furthermore, the kit is for use in medicine. For example, the kit is for use in a method of preventing, treating, monitoring, and/or diagnosing a disease, optionally selected from a malignant disease, a disease associated with the lymphatic system, a disease in a preformed body cavity, and a joint disease, e.g. for use in medical imaging, radioguided surgery, and/or radionuclide therapy, preferably in scintigraphy such as lymphoscintigraphy, single-photon emission computed tomography (SPECT), positron emission tomography (PET), and/or in radiosynovectomy.

In one embodiment, said bulking agent is selected from hydroxyethyl starch, trehalose, mannitol, lactose, glycine, Na$_2$CO$_3$, and any combination thereof. In one embodiment, said buffer comprises any of phosphate, TRIS HCl, citrate, histidine, and any combination thereof. In one embodiment, said stabilizing agent comprises any of sucrose, lactose, glucose, trehalose, glycerol, maltose, fructose, mannitol, sorbitol, glycine, alanine, lysine, PEG, dextran, polyvinylpyrrolidone, poloxamer, tween, polyvinyl alcohol, and any combination thereof. In one embodiment, said tonicity regulating agent comprises any of mannitol, sucrose, glycine, glycerol, NaCl, and any combination thereof. In one embodiment, said kit comprises instructions for administration of the nanoparticle.

The use of nanoparticle of the invention, e.g. a nanoparticle comprising block copolymer methoxy poly(ethylene glycol)-poly(lactic-co-glycolic acid) (MeO-PEG-PLGA) and optionally with a poly(lactic-co-glycolic acid) (PLGA)-stabilized core, offers a promising alternative approach to the current radiotracers. PLGA and PEG are biocompatible polymers and already approved by the Food and Drug Administration (FDA). They are not categorized as blood products, making their handling significantly easier compared to [$^{99m}$Tc]Tc-HSA colloids. Furthermore, both polymers are completely eliminated from the body. PLGA is biodegraded in aqueous medium to lactic and glycolic acids, which are then further metabolized to carbon dioxide and water and eliminated through the kidneys. PEG, in turn, is excreted unchanged *via* the kidneys. The nanoparticles of the invention comprise pharmaceutically acceptable polymers and the polymers are degraded and/or excreted. Using MeO-PEG-PLGA block copolymer as material for the NPs leads to a fast uptake into the lymphatic system and sentinel lymph nodes, since the PEG layer on the NP surface reduces interaction with the interstitium at the injection site.

The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by $\pm 20\%$, $\pm 15\%$, $\pm 10\%$, and for example $\pm 5\%$. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

The nanoparticles of the invention, e.g. radiolabeled polymer nanoparticles, can be used for medical imaging such as for imaging lymphatic drainage (including the localization of the sentinel lymph node in various tumors and diseases of the lymphatic system), and for imaging the mononuclear phagocyte system (including imaging of the liver, spleen, bone marrow and macrophages e.g. during inflammation and infection) as well as for the treatment of diseases by irradiation with emitted particle radiation. The nanoparticles, preferably made of biodegradable and biocompatible polymers, can be produced in a defined size range in accordance with GMP using a microfluidics-based method. Subsequent labeling, e.g. with technetium-99m, is possible without the use of complexing agents, provided reaction conditions according to the invention are maintained. The nanoparticle of the invention is a new diagnostic agent for medical imaging, particularly for imaging lymphatic drainage with SLN localization and for imaging the mononuclear phagocyte system. The nanoparticles of the invention advantageously have a suitable particle size distribution for medical imaging and are not a blood product, thus overcoming the major drawbacks of currently used standard diagnostic agents. For targeted retention in the lymph node or other target cells, the nanoparticle advantageously can be provided with a targeting agent, and the nanoparticle can bind locally to target structures such as receptors and ectoenzymes of cells in situ via ligands, moiety-binding motifs, and enzyme inhibitors.

Advantageously, the nanoparticle of the invention, particularly the labelled nanoparticle, is an ideal detection agent for medical imaging, particularly an ideal SLN detection agent, since it has the following properties: 1) rapid clearance from injection site, 2) selective uptake into the lymphatic system and high retention in sentinel lymph nodes, 3) low uptake in distal lymph nodes, 4) easily detectable signal intensity, and 5) biodegradation/elimination after signal detection. Advantageously, the nanoparticle having these features allows accurate and high-resolution localization of the sentinel lymph node, thereby minimizing surgical exposure and radiation exposure to the patient and staff.

Advantageously, the microfluidics-based method of the invention enables the production of nanoparticles with a defined and narrow size distribution e.g. between 10 and 50 nm, thereby allowing to produce an ideal size range for imaging the lymphatic system while efficiently accumulating in the sentinel lymph node. The method of the invention advantageously provides a GMP-compliant production of nanoparticles. Furthermore, lyophilization allows for long-term storage of the nanoparticles. The nanoparticles of the invention can be produced and labeled in a simple, fast and controllable way. Furthermore, the nanoparticles can be used for medical imaging, e.g. for sentinel lymph node imaging.

Further advantages of the invention are:

1. a controlled production of nanoparticles having a narrow size range which is ideal for lymphatic scintigraphy (10-50 nm).
2. the nanoparticles are not categorized as a blood product; therefore the regulatory requirements are lower than for previous substances.

3. low production costs.

4. labelling e.g. $^{99m}$Tc labeling without complexing agent

5. labelling e.g. $^{99m}$Tc labeling with >99 % radiochemical purity.

6. high stability in vitro.

7. modification with targeting agents e.g. lymph node-targeting or -retarding targeting agents is possible.

8. lyophilization of the nanoparticles and thus prolonged storage is possible.

BRIEF DESCRIPTION OF THE FIGURES

The present invention is now further described by reference to the following figures. All methods mentioned in the figure descriptions below were carried out as described in detail in the examples.

**Figure 1** shows DLS measurements. Size and PDI of MeO-PEG-PLGA nanoparticles (NPs) prepared at an initial polymer concentration of either (A) 10 mg/ml or (B) 20 mg/ml using different total flow rates (TFR) and flow rate ratios (FRR). Red arrows indicate multimodal size distribution. Results represent mean $\pm$ SD of n = 3 measurements.

**Figure 2** shows NP purification. Size and PDI of three batches of particles before and after purification via ultracentrifugation. Results represent mean $\pm$ SD of n = 3 measurements.

**Figure 3** shows sterile filterability of MeO-PEG-PLGA NPs. (A-C) Size distribution of NPs before and after sterile filtration. (D) Ratio of NP size after ($S_f$) and before ($S_i$) filtration of three batches. Results represent mean (A-C) or mean $\pm$ SD (D) of n = 3 measurements.

**Figure 4** shows an amount of [$^{99m}$Tc]Tc-MeO-PEG-PLGA NPs after radiolabelling using 40, 120 or 200 $\mu$g stannous chloride dihydrate at pH 5, 6 or 7. Results represent mean $\pm$ SD of n = 3 experiments.

**Figure 5** shows an effect of cysteine and DTPA on complex stability. Percentage of transchelation after challenge of [99$^m$Tc]Tc-PEG-PLGA NPs with 40 mM cysteine or 10 mM DTPA. Results represent mean $\pm$ SD of n = 3 experiments.

**Figure 6** shows an illustration of the nanoparticle of the invention, particularly radiotracer development. MeO-PEG-PLGA NPs were prepared using microfluidics and subsequently $^{99m}$Tc-labelled by a direct method.

**Figure 7** shows three stages of nanoprecipitation of MeO-PEG-PLGA NPs. Nucleation of monomers (stage I), followed by nuclei growth through aggregation of further polymers to the nuclei (stage II), resulting in kinetically locked NPs with slow exchange of monomers (stage III).

**Figure 8** shows a number-weighted size distribution of MeO-PEG-PLGA NPs. NPs were prepared using microfluidics at a polymer concentration of 20 mg/ml, a FRR of 7:1 and a TFR of 16 ml/min (optimized parameters). Result represent mean $\pm$ SD of n = 3 NP batches.

**Figure 9** shows colloidal stability of NPs at different pH values. Ratio of NP size after ($S_t$) and before ($S_i$) treatment at pH 5, 6 or 7 of three NP batches. Size changes are indicated by a ratio unequal to 1.0. Red arrows indicate multimodal size distribution. Results represent mean $\pm$ SD of n = 3 measurements.

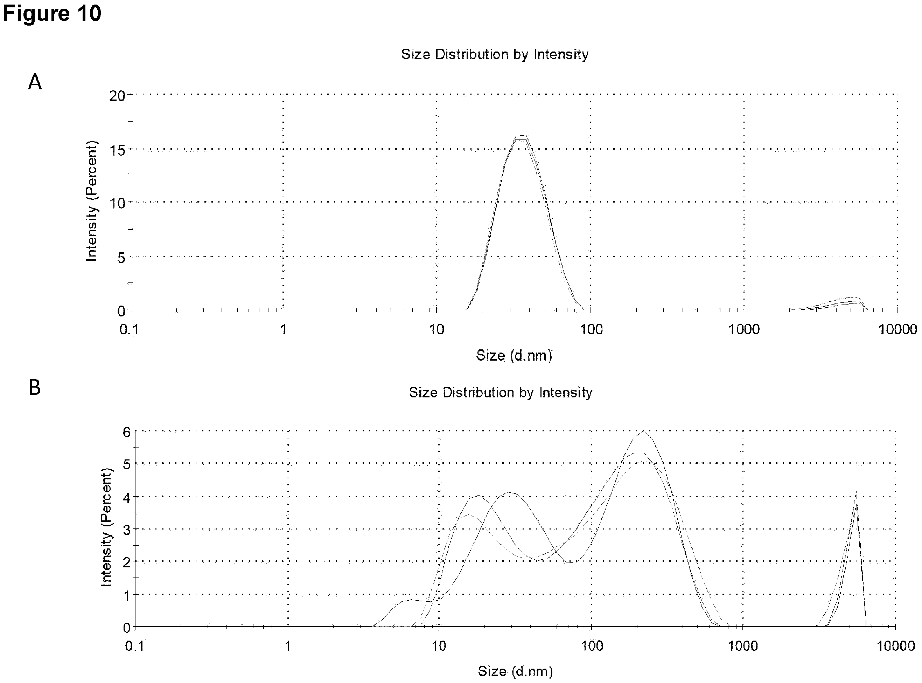

**Figure 10** shows the size distribution of A) labelled nanoparticles of the invention, and B) commercially available Tc-99m-NanoHSA-ROTOP. A) Representative size distribution of $^{99m}$Tc-labelled MeO-PEG-PLGA nanoparticles of the invention. B) Representative size distribution of commercially available Tc-99m-NanoHSA-ROTOP. As shown, the method of the invention allows to provide nanoparticles with an advantageous size distribution profile.

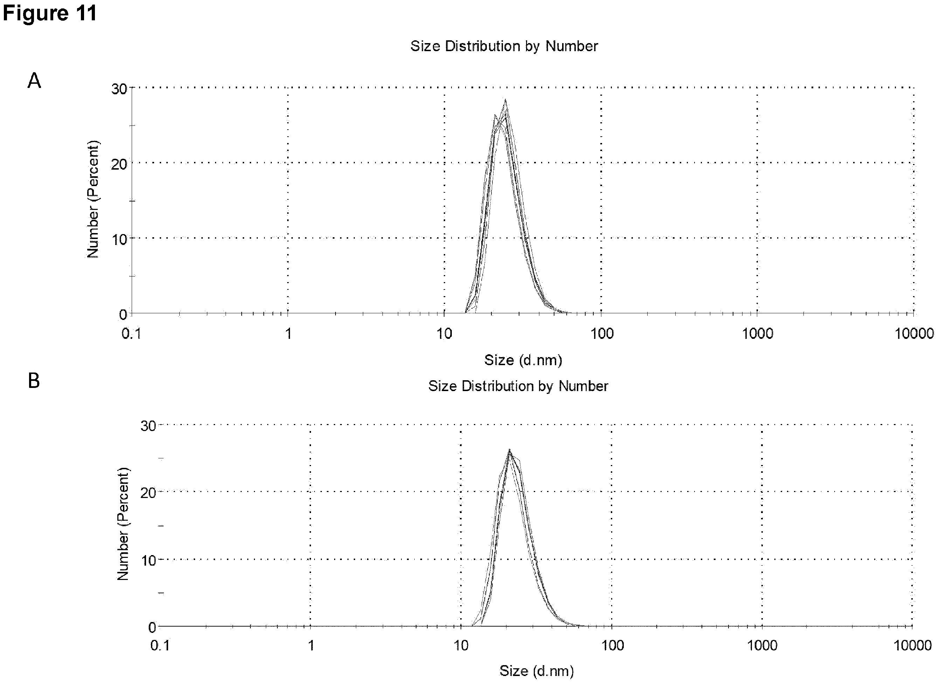

**Figure 11** shows the narrow size distribution of A) MeO-PEG-PLGA nanoparticles of the invention prepared by microfluidics, and B) MeO-PEG-PLGA nanoparticles of the invention prepared by microfluidics and incubated at pH 5. Aggregates are not detectable and the nanoparticles exhibit their original size. In contrast to the size distributions of known radiotracers, the microfluidic method of the invention advantageously produces nanoparticles with a narrow size range between 13.5 and 58.8 nm, with ~85% being smaller than 30 nm. Nanoparticles smaller than 30 nm are both rapidly cleared from the injection site into the lymphatic system and strongly retained in the sentinel lymph node.

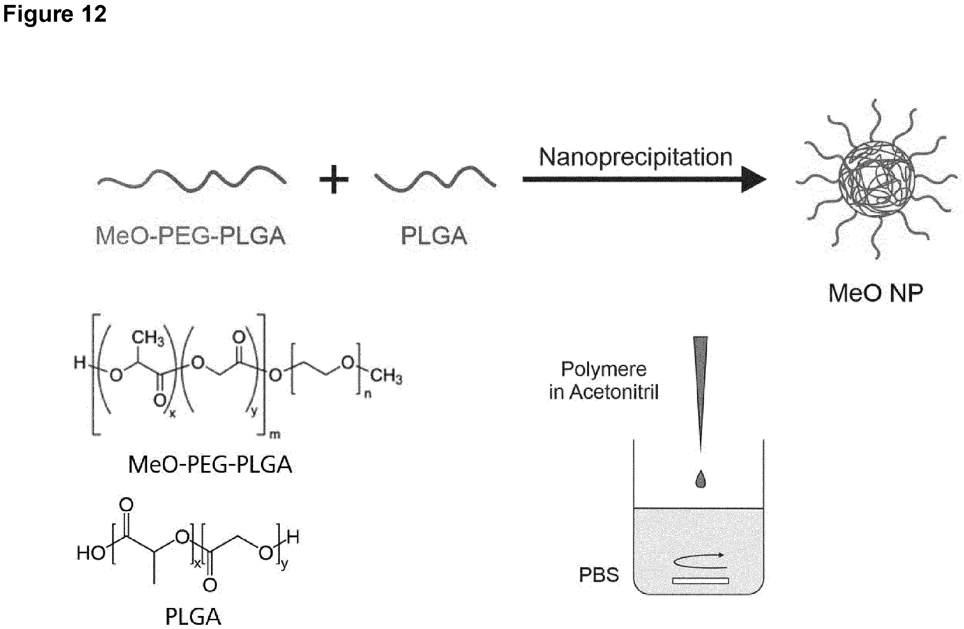

**Figure 12** shows a schematic representation of labelling MeO-PEG-PLGA nanoparticles with $^{99m}$Tc.

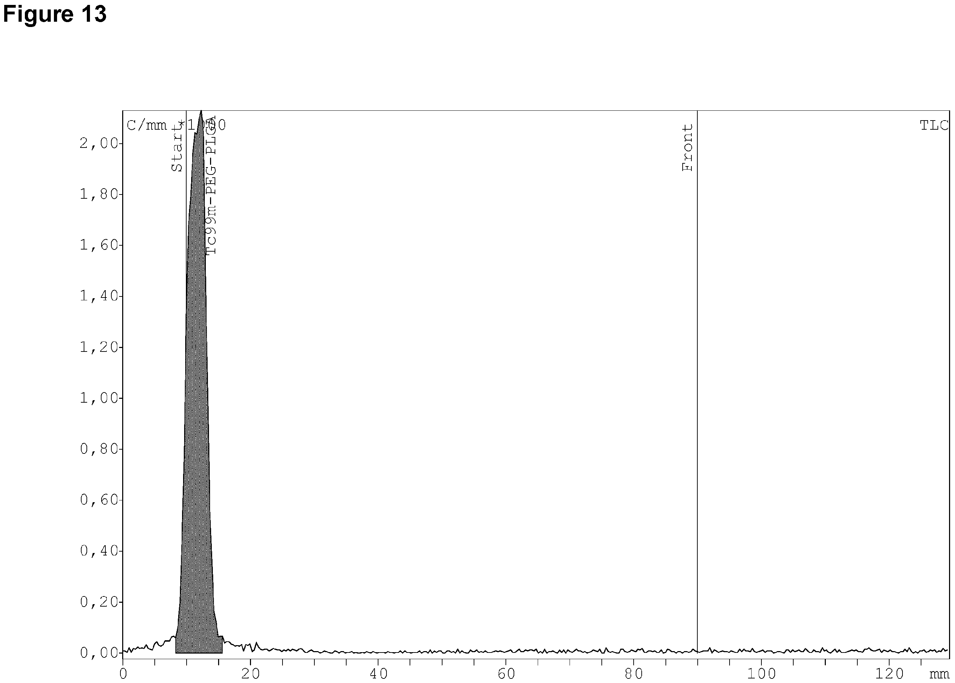

**Figure 13** shows a representative thin-layer chromatogram (ITLC-SA) after labeling under ideal conditions (pH 5, 200 μg Sn(II) chloride). Radio-labeled nanoparticles remain at the base and free sodium pertechnetate would be visible at the front.

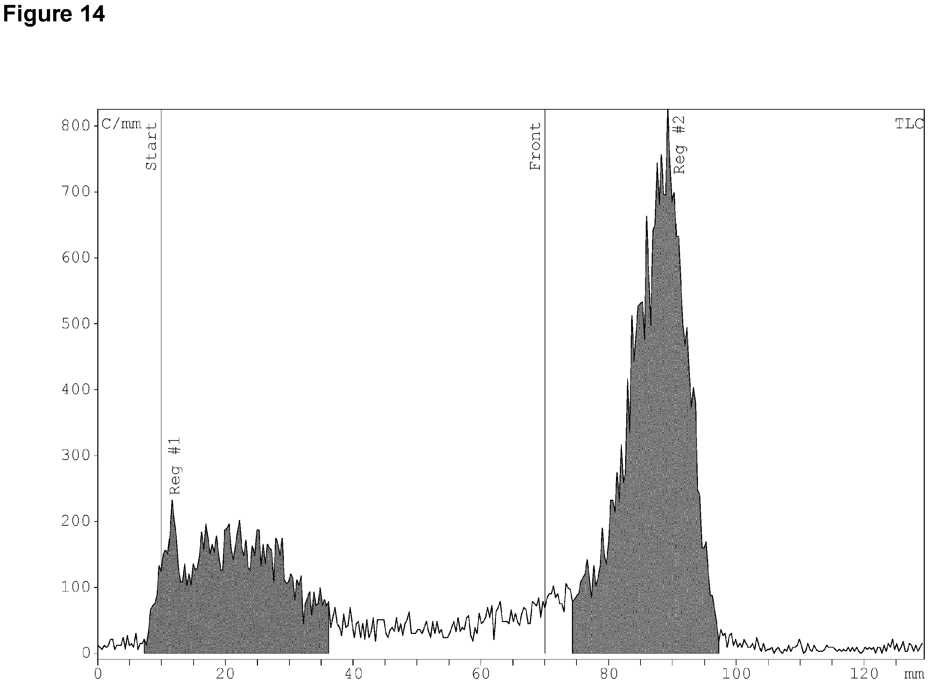

**Figure 14** shows a representative thin-layer chromatogram (ITLC-SG) after labeling without pH adjustment and with 40 μg Sn(II) chloride. Radio-labeled nanoparticles remain at the base and colloids are visible at the front.

In the following, reference is made to the examples, which are given to illustrate, not to limit the present invention.

EXAMPLES

**Example 1: Materials and Methods**

*Materials*

Methoxy-terminated poly(ethylene glycol)-poly(lactic-co-glycolic acid) (MeO-PEG$_{5k}$-PLGA$_{13k}$) with a PEG molecular weight of 5,065 Da and a PLGA molecular weight of 12,771 Da was synthesized by JenKem Technology USA Inc. (Allen, TX, USA). Acetonitrile (for DNA synthesis, max. 10 ppm H$_2$O) and stannous chloride dihydrate (SnCl$_2$·2H$_2$O) was obtained from Merck KGaA (Darmstadt, Germany). L-cysteine hydrochloride monohydrate, diethylenetriaminepentaacetic acid (DTPA) and Gibco™ Dulbecco's phosphate buffered saline (DPBS) were purchased from Thermo Fisher Scientific (Waltham, MA, USA). 0.9% saline and water for injection (WFI) were obtained from B. Braun (Melsungen, Germany). Expansorb® DLG 75-2A (PLGA) and all other materials and reagents were purchased from Sigma Aldrich (Taufkirchen, Germany).

*NP preparation and characterization*

NPs were prepared by microfluidics using the NanoAssemblr™ Benchtop (Precision NanoSystems Inc, Vancouver, Canada). NanoAssemblr™ controller software (v1.09) was used to control the process parameters. The organic phase containing mPEG-PLGA and PLGA in acetonitrile (ACN) at a 7/3 mass ratio was injected into the first inlet port and DPBS as aqueous phase into the second inlet port of the instrument. In order to find optimal instrument and formulation parameters, different total flow rates (TFR), flow rate ratios (FRR) and polymer concentrations were tested. Their influence on particle size and PDI was checked by varying the TFR from 8 to 16 ml/min and FRR (aqueous:organic) from 10:1-1:1 at polymer concentrations of 10 and 20 mg/ml. NP solution was collected and hydrodynamic diameter as well as polydispersity index (PDI) of the freshly prepared undiluted samples were determined by dynamic light scattering (DLS) using a Malvern ZetaSizer Nano ZS (Malvern Instruments GmbH, Lappersdorf, Germany) with a 633 nm He-Ne laser (173° backscatter angle). Measurement was performed at 25 °C in disposable microcuvettes (Brand, Wertheim, Germany).

For all further experiments, NPs were prepared at a TFR of 16 ml/min and a FRR of 7:1 aqueous to organic phase with a polymer concentration of 20 mg/ml. 2 ml of the NP dispersion were collected in an Amicon® Ultra-15 MWCO 100 kDa centrifugal filter (Merck KGaA, Darmstadt, Germany) already containing 2 ml of DPBS. To remove the ACN, NPs were centrifuged in the Amicon® Ultra-15 MWCO 100 kDa centrifugal filter at 1000 g, then diluted with 4 ml DPBS and finally centrifuged again as before. Size and PDI were measured in DPBS (1 mg/ml) as described above.

NP mass concentration was determined as previously described [1]. In brief, PEG content in NPs was quantified using a colorimetric iodine complexing assay [2] and correlated with the exact particle mass determined through lyophilization. NP number concentration $c_N$ was calculated assuming a spherical particle shape after equation 1 [3], where $c_m$ is the particle mass concentration, $\rho_{NP}$ is the density of the NPs (1.3 g/cm$^3$) [4] and $d_{NP}$ is the hydrodynamic diameter of the NPs measured by DLS.

$$c_N = \frac{c_m}{\rho_{NP} \cdot \frac{4}{3}\pi\left(\frac{d_{NP}}{2}\right)^3} \quad (1)$$

*Sterile filtration*

In order to test the sterile filterability of the particles prepared, NP dispersions were sterile filtered through a 0.2 μm Corning® PES syringe filter (Corning Inc., Corning, NY, USA). Particle size and PDI were determined before and after filtration using DLS as described above.

*Colloidal stability at different pH values*

Stability tests were performed to assess whether pH, that needs to be adjusted for labelling reaction, had any effect on colloidal stability of NPs. To this end, 450 $\mu$l of NPs (0.5 mg) were incubated with 450 $\mu$l of 0.9% saline and 100 $\mu$l 10 mM HCl at pH 5, 6 or 7 (adjusted with 0.1N HCl, 1N HCl and 1N NaOH) to mimic later labelling reaction conditions. Afterwards, particle size distribution was determined by DLS as described above.

*$^{99m}$Tc-labelling of NPs and optimization studies*

MeO-PEG-PLGA NPs were radiolabelled with $^{99m}$Tc by a direct method using stannous chloride as reducing agent. Briefly, 0.5 mg NPs (in 450 $\mu$l DPBS) were mixed with 0.1 N HCl, 1 N HCl or 1 N NaOH for pH adjustment. Afterwards, 450 $\mu$l of [$^{99m}$Tc]NaTcO$_4$ solution (0.49-1.01 GBq), eluted with 0.9% saline from a $^{99}$Mo-$^{99m}$Tc generator (Ultra Technekow™ FM, Curium, Petten, Netherlands; Tekcis radionuclide generator, CIS Bio International, Saclay, France), was added to the NP dispersion. Finally, reaction was initiated by adding stannous chloride dihydrate. Labelling was carried out in a TC-ELU-5 vial under nitrogen atmosphere at room temperature for 1.5-3 h. To evaluate the effect of stannous chloride amount and pH on labelling efficiency, various amounts of SnCl$_2$·2H$_2$O (40, 120 and 200 $\mu$g) were tested at different pH values (5, 6 and 7). If not noted otherwise, reaction was performed with 200 $\mu$g SnCl$_2$·2H$_2$O at pH 5.

*Determination of labelling efficiency*

Labelling efficiency was assessed by ascending instant thin layer chromatography (ITLC). Two separate runs were performed for each reaction: For the first run, 1 $\mu$l of the reaction mixture was spotted 1 cm above the bottom of a polysilica acid gel impregnated glass fiber sheet (ITLC-SA; Gelman Sciences Inc., Ann Arbor, MI, USA) and developed with MEK as mobile phase to determine free [$^{99m}$Tc]TcO$_4^-$. For run two, 1 $\mu$l of the reaction mixture was spotted 1 cm above the bottom of a silica gel coated glass microfiber chromatography paper (ITLC-SG, Agilent Technologies, Folsom, CA, USA) and developed with ACD solution (0.068 M citrate, 0.074 M dextrose, pH = 5) to determine hydrolyzed-reduced (H-R) $^{99m}$Tc ([$^{99m}$Tc]Tc-Sn colloid and [$^{99m}$Tc]TcO$_2$). The radioactivity of the sheets was measured with a MiniGITA radiometric TLC scanner (Elysia-raytest GmbH, Straubenhardt, Germany). When using MEK as mobile phase, free [$^{99m}$Tc]TcO$_4^-$ migrated to the top of the ITLC strip and labelled NPs as well as radiocolloids (H-R $^{99m}$Tc) remained at the application point. On the other hand, when using ACD solution, free [$^{99m}$Tc]TcO$_4^-$ and H-R $^{99m}$Tc migrated with the solvent front, while labelled NPs remained at the beginning. Citric acid in the ACD solution is a weak complexing agent, so can solubilize the radiocolloids, causing them to migrate with the mobile phase. However, the [$^{99m}$Tc]Tc-NP complexes are stronger and remain intact and are thus too large to migrate and therefore remain at the starting point. The amount of free [$^{99m}$Tc]TcO$_4^-$ corresponds to the amount (%) migrated in MEK, the amount of radiocolloids was calculated by subtracting the percentage migrated in MEK from the percentage migrated in ACD. The amount of labelled NPs (= labelling efficiency) corresponds to the amount (%) not migrated in ACD.

*DTPA and cysteine challenge*

In order to check the binding strength of the NPs with $^{99m}$Tc, the radiolabelled NPs were challenged with DTPA and cysteine. In short, solutions of 10 mM DTPA or 40 mM cysteine in WFI were prepared. 200 - 300 $\mu$l labelled NPs were mixed with 500 $\mu$l DTPA and cysteine solution, respectively. After 1.5-6 h incubation at room temperature, the effect of DTPA and cysteine on labelling efficiency was evaluated by ITLC with ACD solution as mobile phase, as described above. In this system, free [$^{99m}$Tc]TcO$_4^-$ and all known chemical forms of [$^{99m}$Tc]Tc-DPTA and [$^{99m}$Tc]Tc-cysteine migrated upward and labelled NPs remained at the point of application.

**Example 2: Results**

*Formulation optimization studies*

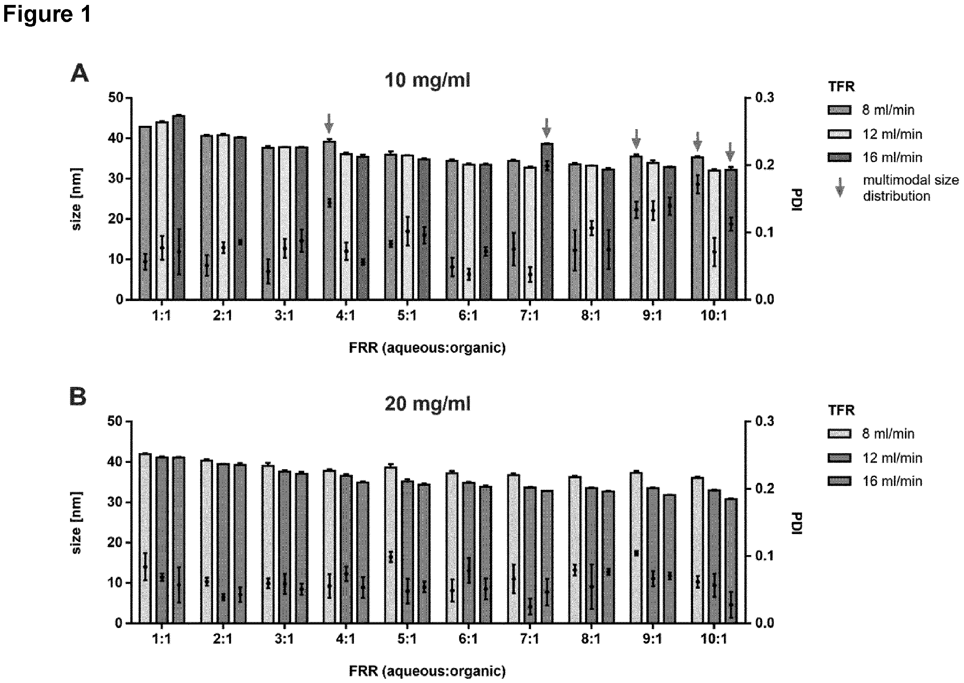

For the manufacturing of the nanoparticles, e.g. MeO-PEG-PLGA NPs, the inventors used microfluidics. Polymers dissolved in ACN (organic phase) and DPBS (aqueous phase) were injected in the microfluidic cartridge of the NanoAssemblr™ Benchtop and the NP preparation was optimized by changing TFR, FRR and polymer concentration. As displayed in Figure 1, polymer concentration had minimal effect on particle size. The hydrodynamic diameter of the NPs was 32.0-45.5 nm at 10 mg/ml (Figure 1A) and 30.8-42.0 nm at 20 mg/ml (Figure 1B). A higher polymer concentration thus resulted in minimally smaller particles. Furthermore, the NPs prepared at 10 mg/ml showed a multimodal size distribution with aggregates in five approaches, while particles manufactured at 20 mg/ml always had a unimodal size distribution. This is also reflected in the higher PDI (0.037-0.199) for the lower concentration compared to the PDI of 0.025-0.104 for the higher concentration.

Regarding the different FRRs, a decrease in particle size with increasing aqueous fraction was detected at both polymer concentrations.

Finally, for almost all polymer concentration-FRR combinations, higher TFR resulted in smaller NPs. The batches produced at FRR 1:1 and 10 mg/ml showed an inverse correlation and also the particles prepared at polymer concentration 10 mg/ml, FRR 7:1 and TFR 16 ml/min were out of the line.

Since the approach synthesized at a polymer concentration of 20 mg/ml, a FRR of 7:1 and a TFR of 16 ml/min exhibited a small size (32.9±0.0 nm) and a narrow size distribution (PDI 0.046±0.020) combined with a sufficient particle yield, the inventors decided to use these parameters for future preparations.

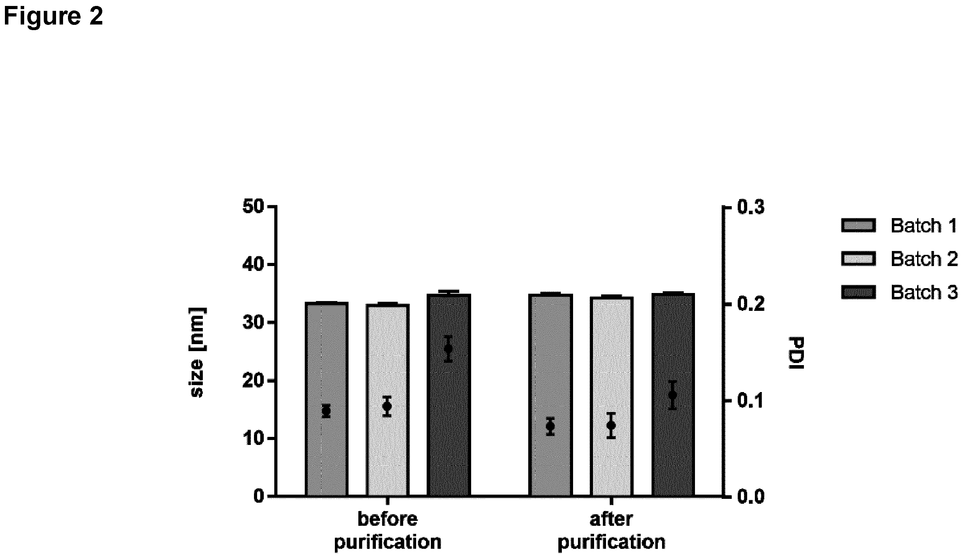

Next, the inventors evaluated whether the washing procedure via ultracentrifugation affects particle size. DLS measurements of three batches revealed no changes in hydrodynamic diameter and a slight decrease in PDI after purification (Figure 2).

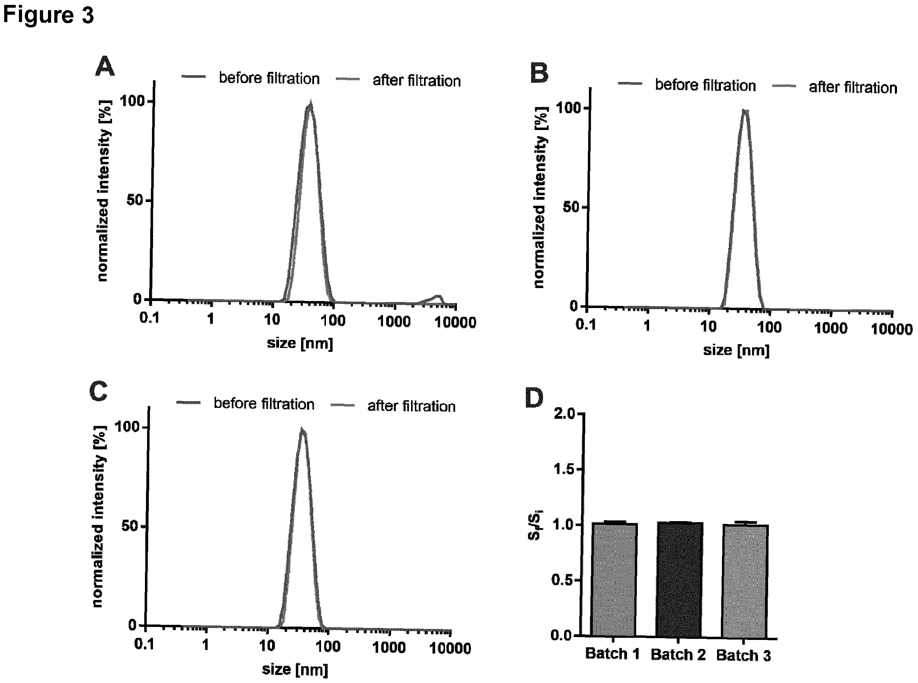

Finally, the inventors investigated the sterile filterability of the NPs by determining the size and size distribution before and after filtration through a 0.2 $\mu$m PES filter. Figure 3 A-C shows that aggregates were removed by filtration and that the particles depicted no changes in size as the particle peaks of the size distribution curves were congruent. Additionally, the post-filtration NP size ($S_f$) relative to the pre-filtration NP size ($S_i$) was approximately 1.0 for all three batches, confirming that sterile filtration did not change size.

### $^{99m}$Tc-labelling optimization studies

The inventors labelled MeO-PEG-PLGA NPs with $^{99m}$Tc by a direct method using stannous chloride as reducing agent. Since the pH and the amount of stannous chloride may strongly affect the formation of radiocolloids (H-R $^{99m}$Tc) and the labelling efficiency, the inventors tested various amounts of $SnCl_2 \cdot 2H_2O$ and different pH values. As shown in Table 1, free $[^{99m}Tc]TcO_4^-$ was only detectable at pH 7 in combination with the lowest stannous chloride amount (40 $\mu$g). No free pertechnetate was found for any of the other approaches.

Table 1. Influence of pH value and amount of stannous chloride on labelling efficiency. Results represent mean $\pm$ SD of n = 3 experiments.

| | 40 $\mu$g $SnCl_2 \cdot 2H_2O$ | | |
| --- | --- | --- | --- |
| | %labelled NPs | %radiocolloids | %free $[^{99m}Tc]TcO_4^-$ |
| pH 5 | 99.1 $\pm$ 1.3 | 0.9 $\pm$ 1.3 | 0.0 $\pm$ 0.0 |
| pH 6 | 43.1 $\pm$ 25.6 | 56.9 $\pm$ 25.6 | 0.0 $\pm$ 0.0 |
| pH 7 | 1.6 $\pm$ 0.8 | 96.8 $\pm$ 2.9 | 1.6 $\pm$ 2.3 |
| | 120 $\mu$g $SnCl_2 \cdot 2H_2O$ | | |
| | %labelled NPs | %radiocolloids | %free $[^{99m}Tc]TcO_4^-$ |
| pH 5 | 72.7 $\pm$ 19.5 | 27.3 $\pm$ 19.5 | 0.0 $\pm$ 0.0 |
| pH 6 | 38.8 $\pm$ 40.3 | 61.2 $\pm$ 40.3 | 0.0 $\pm$ 0.0 |
| pH 7 | 10.7 $\pm$ 7.4 | 89.3 $\pm$ 7.4 | 0.0 $\pm$ 0.0 |
| | 200 $\mu$g $SnCl_2 \cdot 2H_2O$ | | |
| | %labelled NPs | %radiocolloids | %free $[^{99m}Tc]TcO_4^-$ |
| pH 5 | 100.0 $\pm$ 0.0 | 0.0 $\pm$ 0.0 | 0.0 $\pm$ 0.0 |
| pH 6 | 82.9 $\pm$ 18.2 | 17.1 $\pm$ 18.2 | 0.0 $\pm$ 0.0 |
| pH 7 | 23.0 $\pm$ 20.7 | 77.0 $\pm$ 20.7 | 0.0 $\pm$ 0.0 |

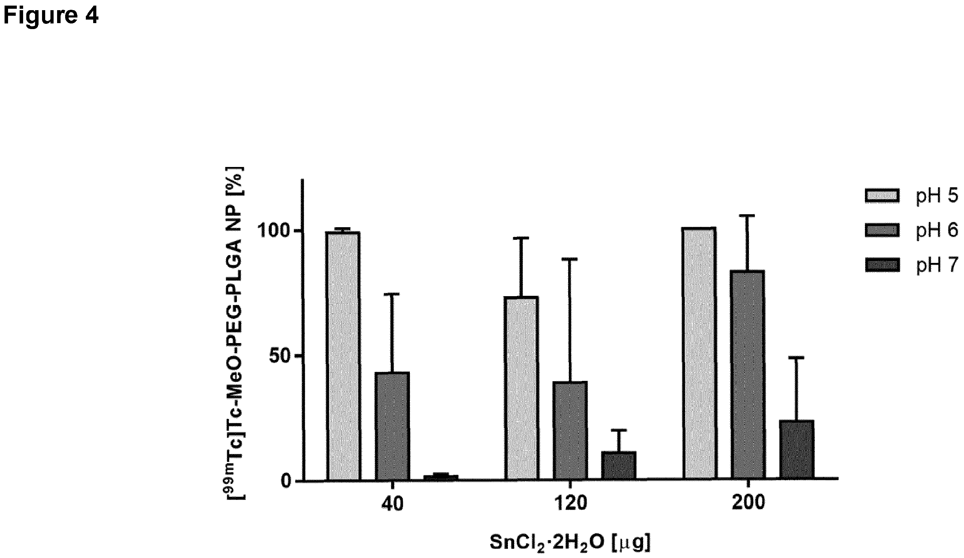

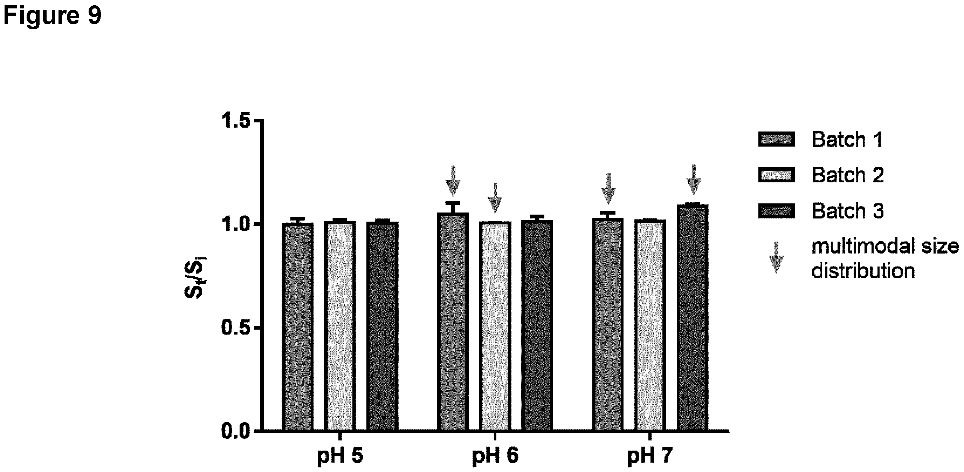

Additionally, the inventors observed that higher pH values and also lower amounts of $SnCl_2 \cdot 2H_2O$ led to the formation of more H-R $^{99m}$Tc and thus poorer labelling efficiency (Table 1 and Figure 4). The optimum reaction conditions resulting in > 99 % radiochemical purity were found to be pH 5 and 200 $\mu$g stannous chloride dihydrate. Finally, pH stability tests revealed that the NPs were most stable at pH 5, since the ratio of NP size after and before treatment was unequal to 1.0 at pH 6 and 7 (Table 2 and Figure 9). At pH 5 the size ratio was 1.0 and no aggregates were visible in all three batches.

**Table 2.** Determined pH in stability studies. Results represent mean $\pm$ SD of n = 3 experiments.

| pH 5 | pH 6 | pH 7 |
|---|---|---|
| 5.09 $\pm$ 0.07 | 6.09 $\pm$ 0.02 | 6.90 $\pm$ 0.02 |

**Table 3. Determined pH** in labelling experiments. Results represent mean $\pm$ SD of n = 3 experiments.

| | 40 $\mu$g SnCl$_2$ | 120 $\mu$g SnCl$_2$ | 200 $\mu$g SnCl$_2$ |
|---|---|---|---|
| pH 5 | 5.15 $\pm$ 0.21 | 5.55 $\pm$ 0.29 | 4.71 $\pm$ 0.23 |
| pH 6 | 6.11 $\pm$ 0.07 | 6.23 $\pm$ 0.11 | 5.99 $\pm$ 0.09 |
| pH 7 | 6.86 $\pm$ 0.04 | 6.99 $\pm$ 0.06 | 6.85 $\pm$ 0.05 |

### *Stability of labelled NPs*

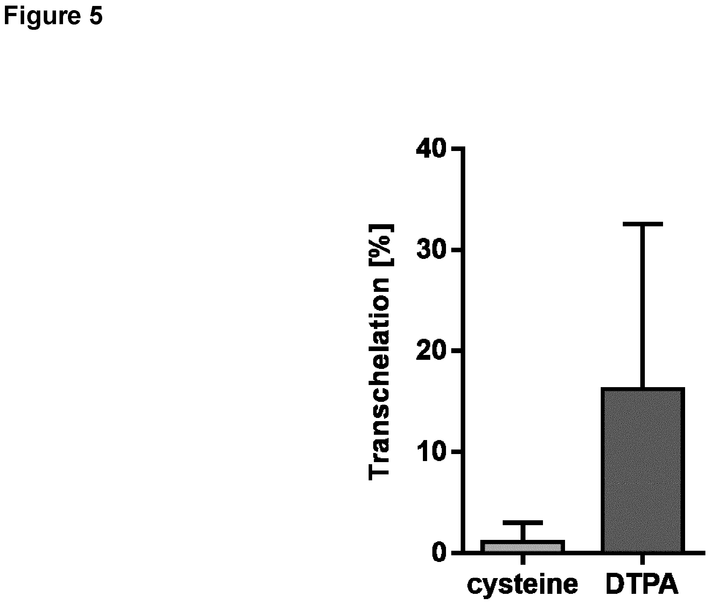

To obtain information about the transchelation of the [99mTc]-labelled NPs, which is a measure for the strength of the complex bond, the inventors performed DTPA and cysteine challenge studies. As shown in Figure 5, cysteine did not alter much the labelling efficiency of the radiolabelled particles (1% transchelation). In the presence of DTPA, in turn, higher transchelation was observed (16%).

### Example 3: Discussion

SLN biopsy is the gold standard in diagnosis and therapy decisions for a variety of tumors. In Germany alone, the number of probe-based SLN detections doubled between 2009 and 2015. Despite this immense need, the currently used and approved radiotracers have many disadvantages. For example, the [99mTc]Tc-HAS, most commonly used in Europe, is categorized as a blood product, which requires an immense effort in documentation and information. The [99mTc]Tc-sulfur colloid, which is approved in the USA, has a very wide size distribution, resulting in bad accuracy of SLN imaging. Finally, the newest product [99mTc]Tc-Tilamanocept is perfect for imaging because with its size of 7 nm it drains very quickly from the injection site into the lymphatic system and due to the integrated mannose ligand it is strongly retained in the SLNs, however, it is extremely expensive and DTPA as a chelator is required.

The invention provides a new radiotracer, particularly [99mTc]-labelled MeO-PEG-PLGA NPs, that is ideally suited for SLN biopsy and represents a promising alternative to currently approved detection agents. Advantageously, the inventors manufactured particles with a mean diameter of about 33 nm and a narrow size distribution using microfluidics. Additionally, the inventors optimized the labelling reaction with [99mTc] in terms of stannous chloride amount and pH, resulting in a product with >99 % radiochemical purity. Finally, the inventors showed that the [99mTc]Tc-NP complex has good stability towards cysteine, which indicates very good complex stability in *vivo.*

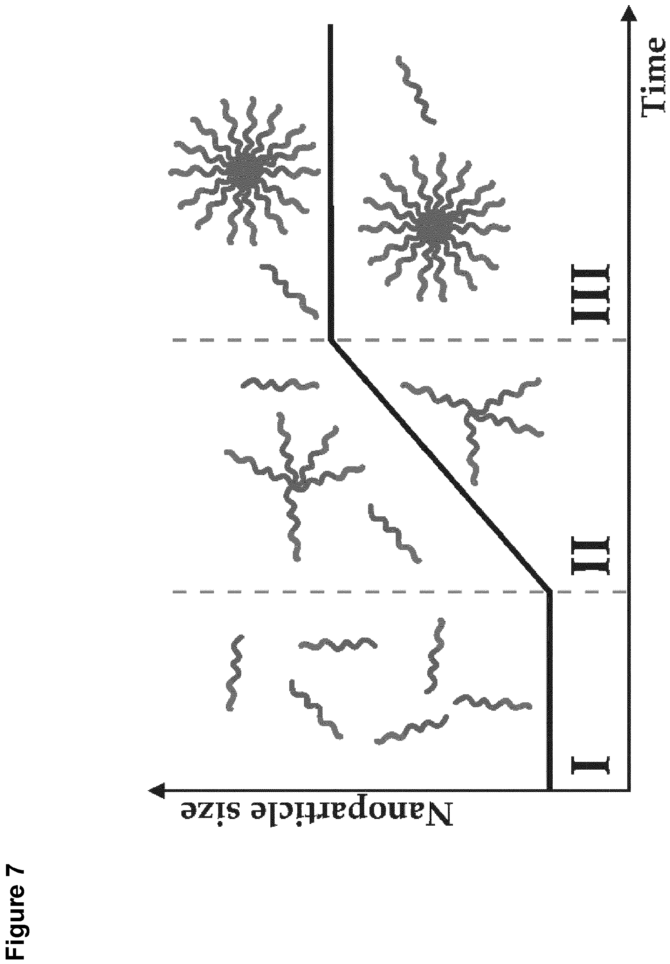

The inventors used MeO-PEG-PLGA block copolymer and PLGA as materials for the carrier NPs because, advantageously, they are excellently biocompatible, biodegradable, non-toxic and moreover, they are not categorized as blood product. The inventors prepared the NPs by nanoprecipitation using a microfluidic device (NanoAssemblr™ Benchtop). Additional PLGA was mixed with the PEG-PLGA polymers, forming a hydrophobic NP core to avoid disassembly in aqueous media. Polymers dissolved in ACN, a water-miscible organic solvent, and DPBS as antisolvent were injected into the microfluidic cartridge. Advantageously, the small dimensions of the microchannels allowed rapid mixing of organic and aqueous phase through diffusion, resulting in reduced solubility and thus supersaturation of the polymers, whereupon they self-assemble into NPs. The particle formation process takes place in three stages (Figure 7). First, single polymers form nuclei due to altered solubility after phase mixing. In the second stage, the nuclei grow through aggregation of further monomers to the nuclei. The final stage III is reached when NPs are sufficiently large and no more unimers can aggregate to the NPs since a polymer brush layer has formed on the surface. The particles are kinetically locked. During the third stage, the NPs are in an equilibrium where only a slow exchange of single polymers occurs without any change in size.

The final size of the NPs strongly depends on the mixing time/flow rate of the organic and aqueous phases. The faster the mixing, the more homogenous the supersaturation of the polymers and the more polymer nuclei are formed initially, resulting in more and smaller NPs. Slower mixing, in turn, results in the local presence of higher levels of organic solvent, leading to poorer nucleation and hence growth of larger particles. Additionally, the degree of supersaturation affects NP size. Higher supersaturation leads to smaller particles since more nuclei precipitate in stage I. On the other hand, at low supersaturation, less nuclei appear initially, increasing the final NP size.

In order to synthesize ideal carriers for SLN biopsy with small size and low polydispersity, the inventors optimized particle preparation by changing instrument parameters such as TFR and FRR and testing different polymer concentrations. Generally, the inventors observed a decrease in particle sizes with higher TFR. Total flow rate directly affects mixing time of organic and aqueous phases. Higher TFRs ensures faster mixing leading to smaller particles as discussed above. Furthermore, influence of FRR on NP size was detectable, as higher aqueous fraction resulted in smaller particles. With higher ratios of nonsolvent to solvent, polymer solubility in the final mixture decreases, resulting in a high supersaturation.

As already explained, high supersaturation leads to smaller particles. Furthermore, high organic solvent fraction facilitates the adsorption of polymers on the NP surface or their insertion into the particles, leading to an increase in size. In contrast to previous studies, that reported larger particle diameters at higher polymer concentrations, the inventors observed an inverse trend with decreasing size at higher concentrations. Without wishing to be bound by any theory, the inventors think that the higher polymer concentration led to higher supersaturation and hence smaller particles. In addition, the inventors saw an increase in PDI at lower concentrations, indicating that the low supersaturation led to secondary nucleation resulting in a broader size distribution.

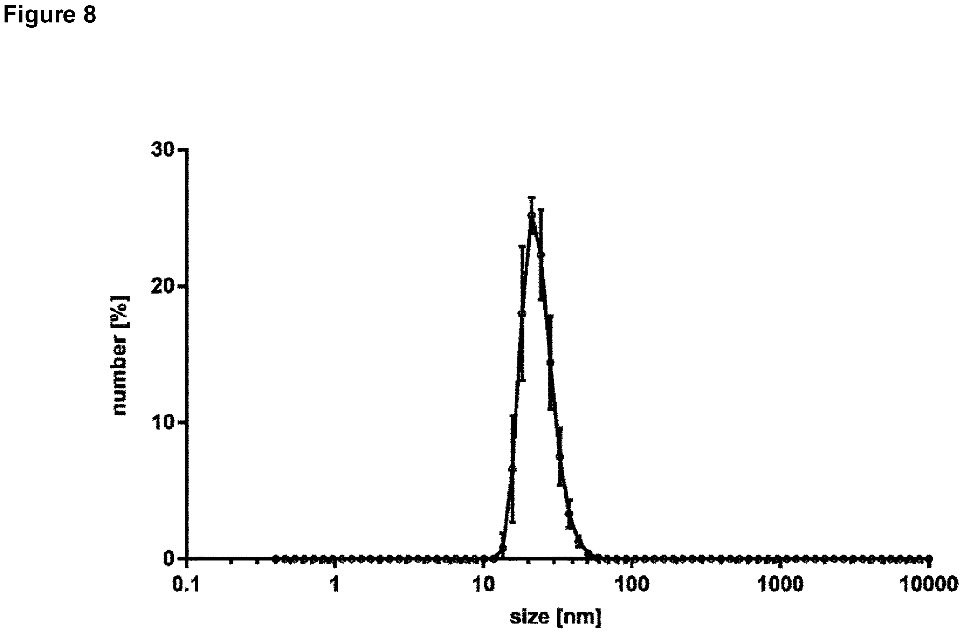

NPs prepared at polymer concentration of 20 mg/ml, FRR of 7:1 and TFR of 16 ml/min depicted the best properties in terms of size and PDI for later use as radiotracers. Their mean size was about 33 nm (Figure 8). Typically, PEG-PLGA NPs with a diameter of about or below 33 nm are effectively drained to the lymph nodes. The particles of the invention therefore have ideal dimensions for SLN detection. The narrow size distribution, reflected in the PDI around 0.05, advantageously provides high accuracy in SLN imaging. The particle sizes were between 13.5 and 58.8 nm, with 85% being below 30 nm. Thus, the NPs are not likely to drain to distal lymph nodes since they are larger than 10 nm, nor do they remain at the injection site or in the lymph ducts, but presumably accumulate rapidly in SLNs since the majority (99.5 %) is smaller than 50 nm.

Moreover, the unchanged particle size before and after ultracentrifugation showed that this purification method is suitable for the particles. The decrease in PDI suggests that remaining polymers and micelles were removed by washing.

Finally, the inventors examined the sterile-filterability of the particles. For later clinical use, the NPs has to meet the pharmacopoeial sterility requirements. However, conventional sterilization by autoclaving, heat, gases or $\gamma$-irradiation is not favorable for polymer NPs due to instability and toxicity aspects. The results showed that the particle size did not change through the filtration process. This indicates that filtration through a 0.22 $\mu$m membrane filter is well suited to sterilize these particles.

MeO-PEG-PLGA NPs were directly labelled with $^{99m}$Tc using stannous chloride as reducing agent without the need for a chelating agent. [$^{99m}$Tc]TcO$_4^-$ was reduced from the heptavalent oxidation state to a lower valence state, which then formed a complex with carboxyl and carbonyl groups of PLGA in the NP core. Efficiency of labelling reaction is influenced by the amount of SnCl$_2$·2H$_2$O and the pH. The method of the invention prevented the formation of radiocolloids (H-R $^{99m}$Tc) and residual free pertechnetate. Free pertechnetate is unfavorable because, after s.c. administration, it distributes throughout the blood vessels and interstitium, thus reducing accuracy of SLN imaging. Radiocolloids, on the other hand, are typically unwanted by-products as their size negatively affects the narrow size distribution of NPs and therefore the accuracy of SLN detection. By testing different stannous chloride amounts and pH values, the inventors found optimal reaction conditions where neither free [$^{99m}$Tc]TcO$_4^-$ nor radiocolloids were present. At pH 5 and 200 $\mu$g SnCl$_2$·2H$_2$O, radiochemical purity was >99 %, which will most likely lead to very high accuracy in SLN biopsy. The successful labelling of polymeric NPs by a direct method has been demonstrated by the inventors. Surprisingly, the inventors were able to optimize the reaction to achieve >99 % labelling efficiency. Here the NPs of the invention are superior to the reported approaches as they can be easily $^{99m}$Tc-labelled with maximum efficiency. Additionally, the excellent radiochemical purity allows it to be used without further purification, which is an advantage for the later clinical use.

The stability of the labelled NPs is of immense importance for later application, because in *vivo* decomposition negatively affects the biodistribution of radioactivity and thus the accuracy of SLN biopsy. Since thiols are ubiquitous in proteins, cysteines and glutathione, they are the main competing ligand for $^{99m}$Tc in *vivo*. Thus, the lack of transchelation after cysteine challenge of the $^{99m}$Tc-labelled NPs suggests good complex stability of the developed radiotracer in physiological environments. Transchelation after DTPA challenge is of minor importance as it only indicates that the complex is weaker than a [$^{99m}$Tc]Tc-DTPA complex.

Conclusion

In summary, the inventors provide polymeric NPs that are ideally sized for drainage in the lymph system and retention in SLNs and have a narrow size distribution promising high accuracy of SLN imaging. In addition, the method of the invention advantageously allows $^{99m}$Tc-labelling without a chelating agent and with >99 % radiochemical purity. The minimal transchelation in the presence of cysteine suggests that the tracer is very stable in *vivo*. Apart from the advantages already mentioned, the nanoparticles of the invention, e.g. MeO-PEG-PLGA NPs, offer a number of further advantages. The polymer NPs can be easily lyophilized, which offers the possibility for providing a kit where the unlabelled carrier can be stored in its lyophilized form for a long time and radioactive labelling is carried out just before use. Moreover, a targeting agent, e.g. lymph node-targeting ligand or tumor targeting agent, can be integrated to further enhance SLN retention. In conclusion, the nanoparticles of the are a very promising radioactive tracer and SLN detection agent that may overcome all drawbacks of the current products.

REFERENCES

[1] Abstiens K, Gregoritza M, Goepferich AM. Ligand Density and Linker Length are Critical Factors for Multivalent Nanoparticle-Receptor Interactions. ACSAppl Mater Interfaces. 2019; 11: 1311-1320.

[2] Childs CE. The determination of polyethylene glycol in gamma globulin solutions. Microchemical Journal. 1975; 20: 190-192.

[3] Wen C-Y, Tang M, Hu J, et al. Determination of the Absolute Number Concentration of Nanoparticles and the Active Affinity Sites on Their Surfaces. Anal Chem. 2016; 88: 10134-10142.

[4] Gholizadeh S, Kamps JAAM, Hennink WE, Kok RJ. PLGA-PEG nanoparticles for targeted delivery of the mTOR/PI3kinase inhibitor dactolisib to inflamed endothelium. Int J Pharm. 2018; 548: 747-758.

The features of the present invention disclosed in the specification, the claims, and/or in the accompanying figures may, both separately and in any combination thereof, be material for realizing the invention in various forms thereof.

**Claims**

1. Nanoparticle comprising a block copolymer comprising or consisting of a hydrophobic block and a hydrophilic block;

   wherein said nanoparticle comprises a core and a surface, wherein said core comprises said hydrophobic block and wherein said hydrophilic block is oriented towards said surface;
   wherein said hydrophobic block comprises or consists of a hydrophobic polymer, preferably poly(lactic-co-glycolic acid); and
   wherein said hydrophilic block comprises alkoxy polyethylene glycol, preferably selected from $C_1$-$C_4$ alkoxy polyethylene glycol, more preferably methoxy polyethylene glycol.

2. Nanoparticle according to claim 1, wherein said nanoparticle comprises a label, optionally a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{124}$I, $^{125}$I, $^{131}$I, $^{169}$Er, $^{177}$Lu, $^{186}$Re, $^{188}$Re, $^{198}$Au, and any combination thereof; even more preferably $^{99m}$Tc.

3. Nanoparticle according to claim 1 or 2, wherein said block copolymer is a diblock copolymer or triblock copolymer, preferably a diblock copolymer, more preferably a methoxy-poly(ethylene glycol)-block-poly(lactide-co-glycolide).

4. Nanoparticle according to any one of the foregoing claims, wherein said core further comprises a polymer different from said block copolymer, said polymer preferably comprising or consisting of poly(lactic-co-glycolic acid).

5. Nanoparticle according to any one of the foregoing claims, wherein said nanoparticle has a size in a range of from 5 nm to 200 nm, preferably in a range of from 10 nm to 60 nm, more preferably in a range of from 10 nm to 50 nm, even more preferably in a range of from 13.5 nm to 30 nm.

6. Nanoparticle according to any one of the foregoing claims, wherein said nanoparticle comprises a targeting agent, preferably a lymph node targeting agent and/or a tumor targeting agent;
   wherein, preferably, said targeting agent comprises or consists of a ligand, a moiety-binding motif, an enzyme inhibitor, and/or an antigen-binding peptide.

7. Pharmaceutical composition comprising a nanoparticle according to any one of the foregoing claims and a pharmaceutically acceptable excipient.

8. Nanoparticle according to any one of claims 1-6 or pharmaceutical composition according to claim 7 for use in medicine;

   preferably for use in a method of preventing, treating, monitoring, and/or diagnosing a disease, optionally selected from a malignant disease, a disease associated with the lymphatic system, a disease in a preformed body cavity, and a joint disease;
   more preferably for use in medical imaging, radioguided surgery, and/or radionuclide therapy, preferably in scintigraphy such as lymphoscintigraphy, single-photon emission computed tomography (SPECT), positron emission tomography (PET), and/or in radiosynovectomy.

9. Method of preparing a nanoparticle, preferably a nanoparticle according to any one of claims 1-6, comprising

   a) providing a block copolymer comprising or consisting of a hydrophobic block and a hydrophilic block, wherein said hydrophobic block comprises or consists of a hydrophobic polymer, preferably poly(lactic-co-glycolic acid), and wherein said hydrophilic block comprises alkoxy polyethylene glycol, preferably selected from $C_1$-$C_4$ alkoxy polyethylene glycol, more preferably methoxy polyethylene glycol;
   b) dissolving said block copolymer in an organic solvent, preferably selected from acetonitrile, N,N-dimethylformamide, acetone, tetrahydrofuran, dimethyl sulfoxide, and any combination thereof, more preferably acetonitrile, thereby obtaining a polymer solution;
   c) optionally, adding a polymer different from said block copolymer, said polymer preferably comprising or consisting of poly(lactic-co-glycolic acid), to said polymer solution;
   d) contacting, particularly mixing, said polymer solution obtained in step b) or step c) with an aqueous phase, preferably using a microfluidic system;
   e) obtaining nanoparticle(s), preferably by self-assembly, more preferably by nanoprecipitation;
   said nanoparticle(s) optionally having a size in a range of from 5 nm to 200 nm, preferably in a range of from 10 nm to 60 nm, more preferably in a range of from 10 nm to 50 nm, even more preferably in a range of from 13.5 nm to 30 nm;
   f) optionally, lyophilizing said nanoparticle(s) to obtain lyophilized nanoparticle(s).

10. The method according to claim 9, wherein the method comprises step c) of adding a polymer different from said block copolymer, said polymer preferably comprising or consisting of poly(lactic-co-glycolic acid), to said polymer solution;
   wherein, preferably, said block copolymer and said polymer different from said block copolymer have a weight ratio of about 7:3 (m/m).

11. The method according to any one of claims 9-10, wherein said method further comprises labelling, preferably radioactively labelling, said nanoparticle(s) obtained in step e) or said lyophilized nanoparticle(s) obtained in step f); said labelling preferably comprising

   i) providing a label, optionally a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{124}$I, $^{125}$I, $^{131}$I, $^{169}$Er, $^{177}$Lu, $^{186}$Re, $^{188}$Re, $^{198}$Au, and any combination thereof; even more preferably $^{99m}$Tc, preferably $^{99m}$Tc pertechnetate, more preferably a $^{99m}$Tc pertechnetate solution; and mixing said label, preferably $^{99m}$Tc pertechnetate, with said nanoparticle(s) obtained in step e) and/or said lyophilized nanoparticle(s) obtained in step f);
   ii) optionally, adding a reducing agent, preferably $SnCl_2$, thereby obtaining a reduced label, preferably reduced $^{99m}$Tc pertechnetate;
   iii) optionally, adding a stabilizer, preferably ascorbic acid and/or ethanol;
   iv) allowing said label, optionally said reduced label obtained in step ii), preferably reduced $^{99m}$Tc pertechnetate obtained in step ii), to label said nanoparticle(s);
   v) obtaining labelled nanoparticle(s);
   wherein, preferably, said labelling is performed in the absence of oxygen;
   wherein, optionally, said labelling is performed at a pH of about pH 4.5 to about pH 7.5, preferably at a pH of about pH 4.5 to about pH 5.5, more preferably at about pH 5, and/or
   for about 1 min to about 6 hours, preferably about 10 min to about 1.5 hours, more preferably about 10 min to about 20 min, and/or
at a temperature of about 15°C to about 40°C, preferably at room temperature.

12. The method according to claim 11,

wherein said mixing in step i) comprises mixing said label, preferably pertechnetate, with said nanoparticle(s) at a molar ratio of about 1:1, and/or
wherein, if step ii) is present, said adding in step ii) comprises providing a molar ratio of said reducing agent, preferably $SnCl_2$, to said label, preferably pertechnetate, in a range of from about 1000:1 to about 100000:1, optionally in a range of from about 3000:1 to about 30000:1.

13. The method according to any one of claims 11-12, wherein said label, preferably pertechnetate, has a radioactivity, preferably at a time of preparation, in a range of from 4 MBq to 8000 MBq, preferably in a range of from 400 MBq to 2200 MBq, more preferably in a range of from 400 MBq to 1000 MBq.

14. The method according to any one of claims 9-13, wherein said method comprises coupling a targeting agent, preferably a lymph node targeting agent and/or a tumor targeting agent, to said nanoparticle, preferably by coupling said targeting agent to said block copolymer, optionally by coupling said targeting agent to said block copolymer using ethyl(dimethylaminopropyl) carbodiimide and/or N-hydroxysuccinimide.

15. Kit comprising, preferably in separate containers,

- a nanoparticle according to any one of claims 1-6, optionally in a lyophilized form; a pharmaceutical composition according to claim 7; and/or a nanoparticle prepared by a method according to any one of claims 9-14, optionally in a lyophilized form;
- optionally, a label, such as a radioactive label, a fluorescent label, a radioluminescent label, a magnetic label, and/or a dye; preferably a radioactive label; more preferably a radioactive label selected from $^{18}$F, $^{32}$P, $^{44}$Sc, $^{64}$Cu, $^{68}$Ga, $^{89}$Zr, $^{90}$Y, $^{99m}$Tc, $^{111}$In, $^{123}$I, $^{124}$I, $^{125}$I, $^{131}$I, $^{169}$Er, $^{177}$Lu, $^{186}$Re, $^{188}$Re, $^{198}$Au, and any combination thereof; even more preferably $^{99m}$Tc, preferably $^{99m}$Tc pertechnetate, optionally a $^{99m}$Tc pertechnetate solution, e.g. a solution of sodium pertechnetate;
- optionally, a reducing agent, preferably $SnCl_2$; and
- optionally, a bulking agent, a buffer, a cryoprotectant, a stabilizing agent, and/or a tonicity regulating agent.

# Figure 1

EP 4 360 661 A1

Figure 2

EP 4 360 661 A1

Figure 3

Figure 4

EP 4 360 661 A1

Figure 5

# Figure 6

Formulation parameter:
- Polymer concentration

Instrument parameter:
- Total flow rate
- Flow rate ratio
(aqueous:organic)

Reaction conditions:
- stannous chloride amount
- pH value

MeO-PEG-PLGA    PLGA

Polymers in ACN

PBS

NanoAssemblr

MeO-PEG-PLGA NP

99mTc-labelling

[99mTc]Tc-MeO-PEG-PLGA NP

Quality attributes:
- small size (30 nm)
- narrow size distribution

Quality attributes:
- no free [99mTc]TcO$_4^-$
- no radiocolloids
- >99% radiochemical purity

## Figure 7

EP 4 360 661 A1

**Figure 8**

Figure 9

# Figure 10

**A**

Size Distribution by Intensity

**B**

Size Distribution by Intensity

# Figure 11

# Figure 12

EP 4 360 661 A1

Figure 13

EP 4 360 661 A1

**Figure 14**

Europäisches
Patentamt

European
Patent Office

Office européen
des brevets

# EUROPEAN SEARCH REPORT

Application Number

EP 22 20 3328

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HE ZELAI ET AL: "Immune activity and biodistribution of polypeptide K237 and folic acid conjugated amphiphilic PEG-PLGA copolymer nanoparticles radiolabeled with 99mTc", ONCOTARGET, vol. 7, no. 47, 22 November 2016 (2016-11-22), pages 76635-76646, XP093034310, United States ISSN: 1949-2553, DOI: 10.18632/oncotarget.12850 * abstract * * page 76635, column 2, paragraph 2 - page 76636, column 1, paragraph 2 * * page 76641, column 1, paragraph 5 - column 2, paragraph 1 * * page 76641, column 2, paragraph 4 * * scheme 1 * | 1-3,5-9, 11-15 | INV. A61K51/12 A61K103/10 B82Y15/00 A61P19/02 A61P35/00 |
| X | US 5 543 158 A (GREF RUXANDRA [FR] ET AL) 6 August 1996 (1996-08-06) * figure 1 * * examples 1-4, 6 * * column 3, line 55 - column 4, line 2 * | 1-11,14, 15 | TECHNICAL FIELDS SEARCHED (IPC) A61K A61P B82Y |

-/--

The present search report has been drawn up for all claims

| Place of search | Date of completion of the search | Examiner |
|---|---|---|
| The Hague | 27 March 2023 | Monami, Amélie |

EPO FORM 1503 03.82 (P04C01)

Europäisches
Patentamt

European
Patent Office

Office européen
des brevets

# EUROPEAN SEARCH REPORT

Application Number

EP 22 20 3328

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BELETSI A ET AL: "Biodistribution properties of nanoparticles based on mixtures of PLGA with PLGA-PEG diblock copolymers", INTERNATIONAL JOURNAL OF PHARMACEUTICS, ELSEVIER, NL, vol. 298, no. 1, 14 July 2005 (2005-07-14), pages 233-241, XP027623984, ISSN: 0378-5173 [retrieved on 2005-07-14] * abstract * * page 235, column 1, paragraph 2 - column 2, paragraph 1 * * table 1 * | 1-5, 7-10,15 | |
| X | BRENDA L SANCHEZ-GAYTAN ET AL: "Real-Time Monitoring of Nanoparticle Formation by FRET Imaging", ANGEWANDTE CHEMIE INTERNATIONAL EDITION, VERLAG CHEMIE, HOBOKEN, USA, vol. 56, no. 11, 23 January 2017 (2017-01-23), pages 2923-2926, XP072089042, ISSN: 1433-7851, DOI: 10.1002/ANIE.201611288 * page 2924, column 2, paragraph 3 - page 2925, column 1, paragraph 1 * * figure 3 * | 1-5, 7-10,15 | |

-/--

TECHNICAL FIELDS SEARCHED (IPC)

The present search report has been drawn up for all claims

| Place of search | Date of completion of the search | Examiner |
|---|---|---|
| The Hague | 27 March 2023 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 20 3328

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | -& Sanchez-Gaytan Brenda L ET AL: "Supporting Information Real-Time Monitoring of Nanoparticle Formation by FRET Imaging", Angewandte Chemie International Edition, 23 January 2017 (2017-01-23), XP093034326, Retrieved from the Internet: URL:https://onlinelibrary.wiley.com/action/downloadSupplement?doi=10.1002/ange.201611288&file=ange201611288-sup-0001-misc_information.pdf [retrieved on 2023-03-23] * page 3, paragraph 3 * * figure S1(b) * ----- | | |

TECHNICAL FIELDS
SEARCHED      (IPC)

The present search report has been drawn up for all claims

| Place of search | Date of completion of the search | Examiner |
|---|---|---|
| The Hague | 27 March 2023 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 3328

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2023

| Patent document cited in search report | | Publication date | Patent family member(s) | Publication date |
|---|---|---|---|---|
| US 5543158 | A | 06-08-1996 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ABSTIENS K ; GREGORITZA M ; GOEPFERICH AM ; LIGAND DENSITY ; LINKER LENGTH.** Critical Factors for Multivalent Nanoparticle-Receptor Interactions. *ACSAppl Mater Interfaces,* 2019, vol. 11, 1311-1320
- **CHILDS CE.** The determination of polyethylene glycol in gamma globulin solutions. *Microchemical Journal,* 1975, vol. 20, 190-192
- **WEN C-Y ; TANG M ; HU J et al.** Determination of the Absolute Number Concentration of Nanoparticles and the Active Affinity Sites on Their Surfaces. *Anal Chem.,* 2016, vol. 88, 10134-10142
- **GHOLIZADEH S ; KAMPS JAAM ; HENNINK WE ; KOK RJ.** PLGA-PEG nanoparticles for targeted delivery of the mTOR/PI3kinase inhibitor dactolisib to inflamed endothelium. *Int J Pharm,* 2018, vol. 548, 747-758